# EUROPEAN PATENT SPECIFICATION

(11) **EP 4 211 263 B1**
(45) Date of publication and mention of the grant of the patent: **11.12.2024**
(21) Application number: 21791448.0
(22) Date of filing: 13.09.2021
(51) Int. Cl.: C12Q 1/6806

(54) **CYTOSINE MODIFICATION ANALYSIS**
CYTOSINMODIFIKATIONSANALYSE
ANALYSE DE MODIFICATIONS DE CYTOSINE

(30) Priority: 14.09.2020 US 202063078181 P
(43) Date of publication of application: 19.07.2023
(73) Proprietor: Ludwig Institute for Cancer Research Ltd, 8001 Zürich (CH)
(72) Inventor: SONG, Chunxiao, Oxford, Oxfordshire OX1 2JD (GB); LIU, Yibin, Oxford, Oxfordshire OX1 2JD (GB)
(74) Representative: Forresters IP LLP
(86) International application number: PCT/IB2021/000630
(87) International publication number: WO 2022/053872

(56) References cited:
- WO-A1-2014/083118
- WO-A1-2019/136413
- WO-A1-2021/005537
- LIU YIBIN ET AL: "Bisulfite-free direct detection of 5-methylcytosine and 5-hydroxymethylcytosine at base resolution", NATURE BIOTECHNOLOGY, NATURE PUBLISHING GROUP US, NEW YORK, vol. 37, no. 4, 25 February 2019 (2019-02-25), pages 424 - 429, XP036900638, ISSN: 1087-0156, [retrieved on 20190225], DOI: 10.1038/S41587-019-0041-2
- ZENG HU ET AL: "Bisulfite-Free, Nanoscale Analysis of 5-Hydroxymethylcytosine at Single Base Resolution", JOURNAL OF THE AMERICAN CHEMICAL SOCIETY, vol. 140, no. 41, 17 October 2018 (2018-10-17), pages 13190 - 13194, XP055867670, ISSN: 0002-7863, Retrieved from the Internet <URL:https://www.ncbi.nlm.nih.gov/pmc/articles/PMC6423965/pdf/nihms-1017389.pdf> DOI: 10.1021/jacs.8b08297

## Description

### Background

Nucleic acid modifications are an essential part of normal biological function and can play important roles in epigenetic control of gene expression. Improved methods for detection and analysis of cytosine modifications are an area of ongoing research.

WO2019136413A1 provides methods for bisulfite-free identification in a nucleic acid sequence of the locations of 5-methylcytosine, 5-hydroxymethylcytosine, 5-carboxylcytosine and 5-formylcytosine.

LIU YIBIN et al., "Bisulfite-free direct detection of 5-methylcytosine and 5-hydroxymethylcytosine at base resolution", NATURE BIOTECHNOLOGY, NATURE PUBLISHING GROUP US, NEW YORK, (2019) vol. 37, no. 4, pages 424-429 discloses a bisulfite-free and base-level-resolution sequencing method, TET-assisted pyridine borane sequencing (TAPS), for detection of 5mC and ShmC. ZENG HU et al., "Bisulfite-Free, Nanoscale Analysis of 5-Hydroxymethylcytosine at Single Base Resolution", JOURNAL OF THE AMERICAN CHEMICAL SOCIETY, (2018) vol. 140, no. 41, pages 13190 - 13194 discloses hmC-CATCH, a bisulfite-free, base-resolution method for the genome-wide detection of ShmC.

WO2014083118A1 relates to the use of metal (VI) oxo complexes to catalyse the selective oxidation of ShmC residues in polynucleotides to SfC residues.

### Summary

The present invention is defined in the claims. The present disclosure provides technologies for detecting and/or identifying cytosine modifications in nucleic acids, and for analyzing cytosine modifications for clinical applications, such as diagnosis and therapy. In particular, the present disclosure provides technologies for detecting and/or identifying cytosine modification at a single base resolution level. Technologies provided herein do not rely on, and in many embodiments do not utilize, bisulfite treatment. Also, in many embodiments, provided technologies do not require (and/or utilize) enrichment steps that rely on affinity enrichment (e.g., pull-down) technologies.

Liu *et al.* recently described exciting and important technologies for analyzing cytosine modifications, specifically by treating nucleic acids that contain 5-formylcytosine (5fC), and/or 5-carboxylcytosine (ScaC) with borane reducing agents. See, Liu et al., "Bisulfite-free direct detection of 5-methylcytosine and 5-hydroxymethylcytosine at base resolution", Nat. Biotechnol., 37, 2019. Liu *et al.* further demonstrated that such borane reduction technology could advantageously be used together with particular oxidizing technologies (e.g., TET- assisted oxidation and/or metal-oxide treatment), e.g., that can generate the 5fC/5caC-containing nucleic acid, for example by oxidation of SmC and/or ShmC residues in a source nucleic acid.

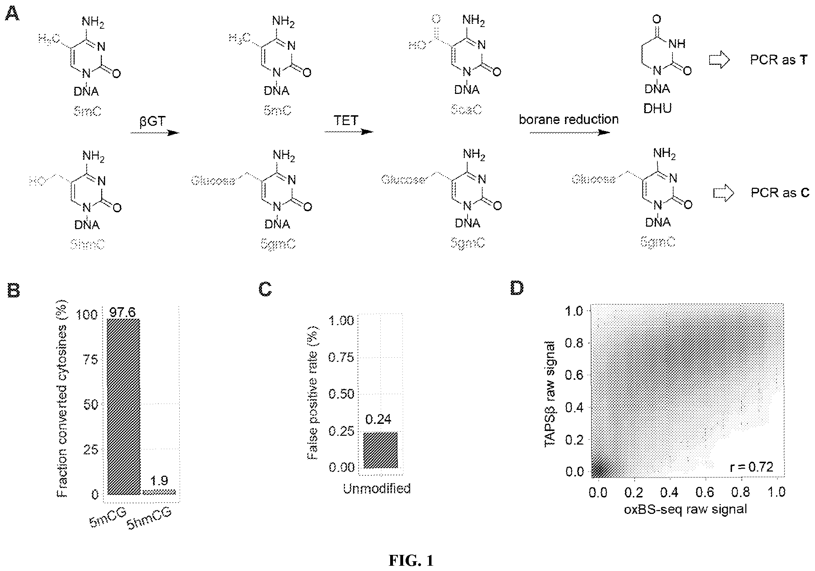

Liu *et al.* provided a suite of nucleic acid processing technologies useful, both individually and in combination, for analysis of cytosine modifications in a nucleic acid of interest. For example, Figure 1 of Liu *et al.* depicts "TET Assisted Pic/Pyridine Borane Sequencing" (TAPS), in which a source nucleic acid is first treated with TET oxidation, followed by borane reduction (preferably with pyridine borane and/or 2-picoline borane (pic-BH3)); TAPSβ, in which the source nucleic acid is first treated with a glucosyltransferase, followed by TET oxidation and subsequent borane reduction (preferably with pyridine borane and/or 2-picoline borane (pic-BH3)); and Chemical Assisted Pic/Pyridine Borane Sequencing, in which the source nucleic acid is first treated with a metal oxide, followed by borane reduction (preferably with pyridine borane and/or 2-picoline borane (pic-BH3)).

TAPS provides an attractive alternative to traditional bisulfite sequencing, as it is nondestructive, detects both SmC and ShmC directly, and displays improved sequence quality, mapping rate, and coverage compared to Bisulfite Sequencing (BS). TAPS and TAPSβ can be run in parallel on samples and subtraction of the assay outputs can indicate the present of ShmC in a sample. Due to the relatively low abundance of ShmC relative to 5mC in most non-neuronal tissues and cell lines, sequence information obtained from subtraction of TAPSβ from TAPS can suffer from high error rates. Subtraction-based methods also generally suffer from increased error rates due to the accumulation of noise from multiple assays, as well as additional considerations for read filtering and statistical analysis (18, 19). TAPSβ and CAPS can be employed as subtraction-free methods to directly measure 5mC and ShmC, respectively, without comparison to another processing technology. Particular advantages of the Liu *et al.* technologies, including the described borane reduction technologies, include mild reaction conditions, easily accessible processing protocols, reduce false positive rates, and high conversion rates, among other things. The present disclosure provides further useful insights relating to, and improvements of, technologies for analyzing cytosine modifications.

In some embodiments, provided technologies are characterized by a low false positive rate, which is particularly surprising when enrichment technologies, such as affinity enrichment (e.g., pull-down technologies) are not employed.

Alternatively, or additionally, in some embodiments, provided technologies are characterized in that some or all steps can be performed without cooling (e.g., at temperatures above 4°C).

Still further alternatively or additionally, in some embodiments, provided technologies achieve efficient detection under mild conditions and/or with streamlined protocols (e.g., with reduced interruption by purification, cleanup and/or enrichment step(s).

Yet further alternatively or additionally, in some embodiments, provided technologies are characterized by low nucleic acid degradation levels.

Provided technologies utilize a metal (VI) oxo complex (e.g., a ruthenate) in a metal oxide oxidation. Provided technologies utilize multiple rounds of oxidation with a metal (VI) oxo complex.

In some embodiments, one or more reactions performed in accordance with the present disclosure are implemented at a temperature above 4°C. In some embodiments, all reactions performed in accordance with the present disclosure are implemented at a temperature above 4°C.

In some embodiments, one or more reactions performed in accordance with the present disclosure are implemented at a pH < 7. In some embodiments, one or more reactions performed in accordance with the present disclosure are implemented at a pH < 6. In some embodiments, a reduction reaction performed in accordance with the present disclosure is implemented at a pH < 6. In some embodiments, all reduction reactions performed in accordance with the present disclosure are implemented at a pH < 6.

In some embodiments, provided technologies do not utilize bisulfite treatment following one or more oxidation steps. In some embodiments, provided technologies do not utilize bisulfite treatment in any step.

In some embodiments, provided technologies do not utilize an enrichment step prior to sequencing. In some embodiments, provided technologies do not utilize an enrichment step prior to borane reduction (e.g., affinity-based enrichment). In some embodiments, provided technologies do not utilize an enrichment step prior to oxidation. In some embodiments, provided technologies do not utilize any enrichment step.

The present disclosure provides a suite of borane reduction technologies that are useful, for example, for direct quantitative sequencing of all four cytosine modifications typically found in nucleic acids of interest (e.g., in mammalian genomic DNA such as genomic stem cell DNA (e.g., mESC or hESC stem cell DNA), tumor DNA, etc).

Among other things, provided technologies offer valuable resources for studying DNA modifications, including in clinical samples and in mammalian model systems (e.g., epigenetics models such as mESCs).

Technologies described herein replace harsh bisulfite treatment with mild borane reduction reaction, and achieve higher sequencing quality and more comprehensive methylome analysis than reported for certain alternative technologies.

Without wishing to be bound by a particular theory, the present disclosure provides an insight that subtraction-free methods for identification and/or detection of modified cytosines offer certain improvements (e.g. increased accuracy, and particularly increased accuracy for low-abundance modifications). In some embodiments, the present disclosure demonstrates that multiple rounds of oxidation of a nucleic acid can, among other things, improve reaction efficiency. In some embodiments, the present disclosure provides technologies for identification and/or detection of modified cytosines with a low false positive rate (e.g., less than 1%). In some embodiments, the present disclosure demonstrates that oxidation and/or reduction of nucleic acid samples can be conducted at room temperature. In some embodiments, the present disclosure demonstrates that provided technologies can be used with minimal degradation of one or more nucleic acid sample(s) (e.g., relative to that observed with other technologies such as, for example, technologies that utilize bisulfite and/or technologies that utilize different oxidation and/or reduction conditions and/or reagents).

Advantages of certain embodiments of provided technologies include that methods may be conducted under mild conditions and/or at room temperature; as documented herein, methods provided by the present disclosure can identify and/or detect modified cytosines with high efficiency and minimal degradation.

### Brief Description of the Drawing

**Fig. 1****. TAPSβ for bisulfite-free 5mC-specific sequencing.** (A) Schematic demonstration of TAPSβ. (B) Conversion rates of TAPSβ at known SmCG or ShmCG positions from CpG-methylated lambda DNA or synthetic spike-in. (C) False-positive rate of TAPSβ from 2 kb-unmodified spike-in. (D) Correlation analysis between TAPSβ and published oxBS-seq dataset at CpGs with the minimal depth of 10. The Pearson's r is shown at the bottom right. The raw signal for each CpG was calculated as the ratio between C and the sum of C and T.
**Fig. 2****. CAPS for bisulfite-free ShmC-specific sequencing.** (A) Schematic demonstration of CAPS. (B) Conversion rates of CAPS at known SmCG or ShmCG positions from CpG-methylated lambda DNA or synthetic spike-in. (C) False positive rate of CAPS from 2kb-unmodified spike-in. (D) Fraction of all sequenced read pairs in CAPS and ACE-seq mapped to the reference mouse genome. (E) Correlation density plot between CAPS, TAB-seq, and ACE-seq in 10-kb bins. (F) Correlation density plot between TAPS-TAPSβ subtraction and TAB-seq or ACE-seq in 10-kb bins.

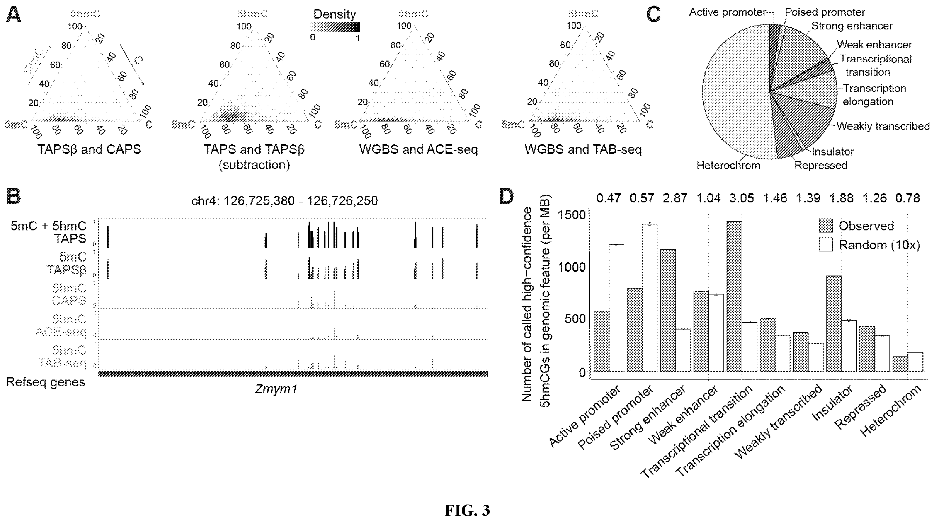

**Fig. 3****. Comparison of CAPS with other methods.** (A) Ternary plots of C, SmC, and ShmC levels tiled by 1-kb bins for TAPSβ, CAPS, ACE-seq and TAB-seq. The levels of unmodified and modified cytosines were estimated by MLML using the direct readout from the method combination shown at the below of each sub figure. (B) Example of genome browser view on chromosome 4 showing CAPS detected consistent ShmC sites when compared with ACE-seq and TAB-seq. (C) Pie chart shows the overlap of called ShmCGs with putative genomic regulatory elements. (D) The relative enrichment of ShmCG (blue) and random sites (white) at genomic regulatory elements. 'Random' consists often random samplings. The mean is shown as the bar height and the error bars denote standard deviation. The ratios between observed and random are shown at the top.
**Fig. 4****. PS for bisulfite-free SfC/ScaC-specific sequencing.** (A) Schematic demonstration of PS and PS-c. (B) Conversion rate of PS at known 5mC, ShmC, 5fC, and ScaC positions in spike-in controls. (C) False-positive rate of PS from 2kb-unmodified spike-in. (D) Conversion rate of PS-c at known 5mC, ShmC, SfC, and ScaC positions in spike-in controls. (E) False-positive rate of PS-c from 2kb-unmodified spike-in.
**Fig. 5****. Base changes in borane reduction chemistry-based methods.** C-toT transitions (marked in red) were recognized as modified sites.
**Fig. 6****. TAPSβ sequencing quality scores per base for the first and second reads in all sequenced read pairs.** Boxplots visualize 10 million random sequencing reads showing medians, upper and lower fourth quantiles and non-outlier extreme values.

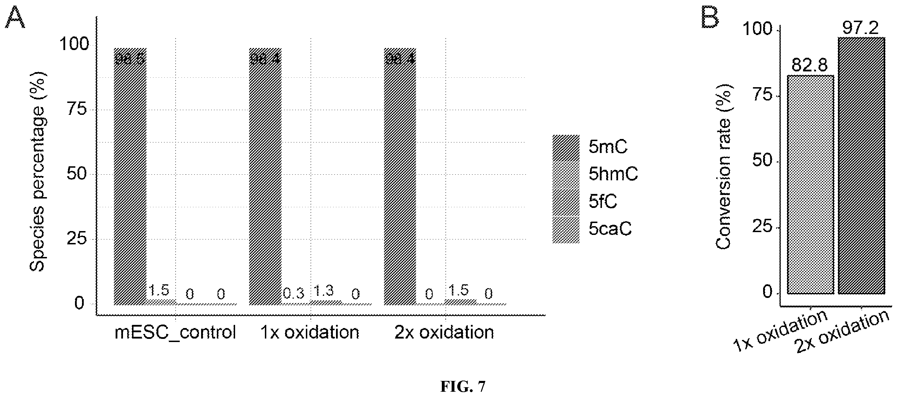

**Fig. 7****. Two rounds of K2RuO4 oxidation achieved more complete 5hmC to 5fC conversion than one round.** (A) HPLC-MS/MS quantification of relative modification levels in the mESCs genomic DNA control, after one round K2RuO4 (1x) oxidation and after two rounds K2RuO4 (2x) oxidation. SfC or ScaC was not detected in the control sample. (B) Conversion rate of ShmC to 5fC was calculated by relative MS signal intensity. Conversion rate = Intensity5fC / (Intensity5fC + Intensity5hmC).

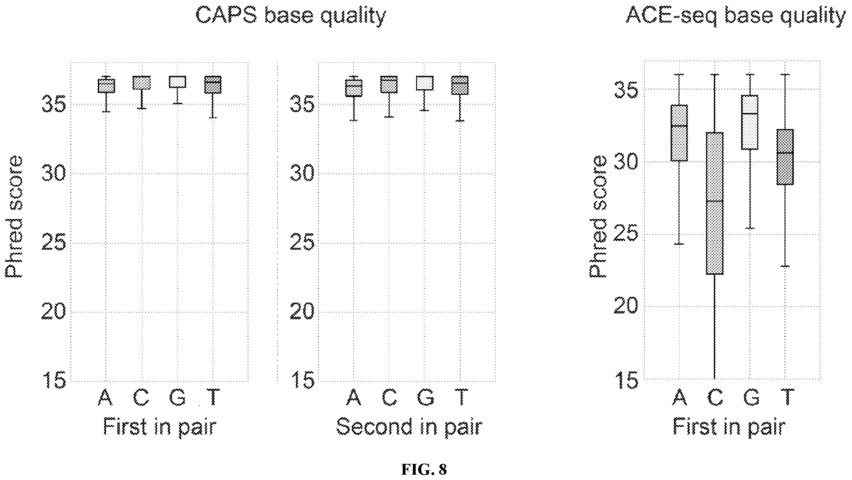

**Fig. 8****. Comparison of sequencing base quality between CAPS** (left) and ACE-seq (right). CAPS (sequenced in pair-end mode) showed good sequencing quality scores per base for the first and second reads in all sequenced read pairs while ACE-seq (sequenced in single-end mode) showed lower sequencing quality scores per base for the first read. Boxplots visualize 10 million random sequencing reads showing medians, upper and lower one-fourth quantiles and non-outlier extreme values.
**Fig. 9****.** Average sequencing coverage depth of CAPS and ACE-seq at all CpG islands (CGI) and 4-kb flanking regions.
**Fig. 10****.** Average sequencing coverage depth of CAPS and ACE-seq at all CpG islands (CGI) and 4-kb flanking regions.
**Fig. 11****.** Conversion rates of unmodified C, SmC, ShmC, SfC, and ScaC in TAPS, TAPSβ, CAPS, PS and PS-c. Conversion rates were calculated based on the corresponding spike-in controls. C: 2kb-unmodified spike-in; 5mC-λ: CpG-methylated lambda DNA; SmC and 5hmC: synthetic spike-in with SmC and ShmC modification; SfC: SfC spike-in; ScaC: ScaC spike-in. Conversion rates of SfC and ScaC are not available (NA) for published TAPS data.
**Fig. 12****.** Alignment and deduplication metrics of sequencing data.

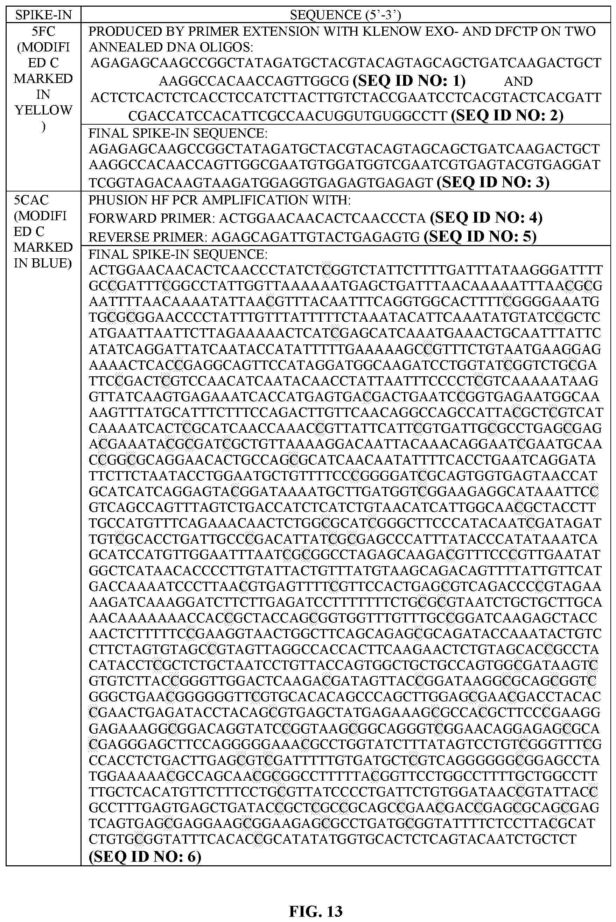

**Fig. 13****.** Primer sequences for SfC and ScaC spike-ins.

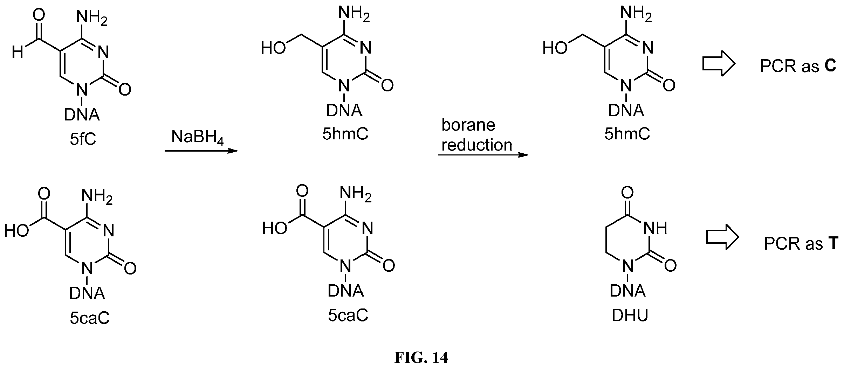

**Fig. 14****.** Schematic demonstration of SfC blocking by NaBH4.

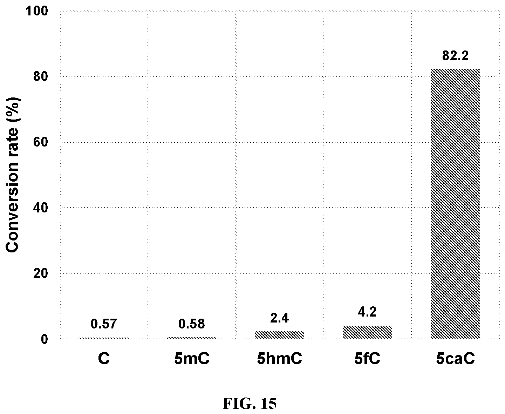

**Fig. 15****.** Conversion rate (percentage) of unmodified and modified cytosines by NaBH4 blocking and pic-borane reaction, measured by high-throughput sequencing with respective spike-in controls.
**Fig. 16****.** Schematic of successive TET oxidation reactions to convert SmC to SmC, SfC, and/or ScaC.

### Definitions

***Biomarker*:** The term "biomarker" is used herein, consistent with its use in the art, to refer to a to an entity, event, or characteristic whose presence, level, degree, type, and/or form, correlates with a particular biological event or state of interest, so that it is considered to be a "marker" of that event or state. To give but a few examples, a biomarker may be or comprise a marker for a particular disease state, or for likelihood that a particular disease, disorder or condition may develop, occur, or reoccur. A biomarker may be or comprise a marker for a particular disease or therapeutic outcome, or likelihood thereof. Thus, a biomarker may be predictive, a biomarker may be prognostic, a biomarker may be diagnostic of the relevant biological event or state of interest. A biomarker may be or comprise an entity of any chemical class, and may be or comprise a combination of entities. For example, a biomarker may be or comprise a nucleic acid, a polypeptide, a lipid, a carbohydrate, a small molecule, an inorganic agent (e.g., a metal or ion), or a combination thereof. A biomarker may be a cell surface marker. A biomarker may be intracellular. A biomarker may be detected outside of cells (e.g., is secreted or is otherwise generated or present outside of cells, e.g., in a body fluid such as blood, urine, tears, saliva, cerebrospinal fluid, etc. A biomarker may be or comprise a genetic or epigenetic signature. A biomarker may be or comprise a gene expression signature.

***Biological Sample*:** As used herein, the term "biological sample" typically refers to a sample obtained or derived from a biological source (e.g., a cell, tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids, secretions, and/or excretions; and/or cells or other components thereof, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, etc.

***Cancer*:** The terms "cancer", "malignancy", "neoplasm", "tumor", and "carcinoma", are used herein to refer to cells that exhibit relatively abnormal, uncontrolled, and/or autonomous growth, so that they exhibit an aberrant growth phenotype characterized by a significant loss of control of cell proliferation. A tumor may be or comprise cells that are precancerous (e.g., benign), malignant, pre-metastatic, metastatic, and/or non-metastatic. The present disclosure specifically identifies certain cancers to which its teachings may be particularly relevant. A relevant cancer may be characterized by a solid tumor. A relevant cancer may be characterized by a hematologic tumor. In general, examples of different types of cancers known in the art include, for example, hematopoietic cancers including leukemias, lymphomas (Hodgkin's and non-Hodgkin's), myelomas and myeloproliferative disorders; sarcomas, melanomas, adenomas, carcinomas of solid tissue, squamous cell carcinomas of the mouth, throat, larynx, and lung, liver cancer, genitourinary cancers such as prostate, cervical, bladder, uterine, and endometrial cancer and renal cell carcinomas, bone cancer, pancreatic cancer, skin cancer, cutaneous or intraocular melanoma, cancer of the endocrine system, cancer of the thyroid gland, cancer of the parathyroid gland, head and neck cancers, breast cancer, gastro-intestinal cancers and nervous system cancers, benign lesions such as papillomas, and the like.

***Chemotherapeutic Agent*:** The term "chemotherapeutic agent", as used herein has its art-understood meaning referring to one or more pro-apoptotic, cytostatic and/or cytotoxic agents, for example specifically including agents utilized and/or recommended for use in treating one or more diseases, disorders or conditions associated with undesirable cell proliferation. Chemotherapeutic agents may be useful in the treatment of cancer. A chemotherapeutic agent may be or comprise one or more alkylating agents, one or more anthracyclines, one or more cytoskeletal disruptors (e.g. microtubule targeting agents such as taxanes, maytansine and analogs thereof, of), one or more epothilones, one or more histone deacetylase inhibitors HDACs), one or more topoisomerase inhibitors (e.g., inhibitors of topoisomerase I and/or topoisomerase II), one or more kinase inhibitors, one or more nucleotide analogs or nucleotide precursor analogs, one or more peptide antibiotics, one or more platinum-based agents, one or more retinoids, one or more vinca alkaloids, and/or one or more analogs of one or more of the following (i.e., that share a relevant anti-proliferative activity). A chemotherapeutic agent may be or comprise one or more of Actinomycin, All-trans retinoic acid, an Auiristatin, Azacitidine, Azathioprine, Bleomycin, Bortezomib, Carboplatin, Capecitabine, Cisplatin, Chlorambucil, Cyclophosphamide, Curcumin, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Maytansine and/or analogs thereof (e.g. DM1) Mechlorethamine, Mercaptopurine, Methotrexate, Mitoxantrone, a Maytansinoid, Oxaliplatin, Paclitaxel, Pemetrexed, Teniposide, Tioguanine, Topotecan, Valrubicin, Vinblastine, Vincristine, Vindesine, Vinorelbine, and combinations thereof. A chemotherapeutic agent may be utilized in the context of an antibody-drug conjugate. A chemotherapeutic agent is one found in an antibody-drug conjugate selected from the group consisting of: hLL1-doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicin, gemtuzumab ozogamicin, brentuximab vedotin, trastuzumab emtansine, inotuzumab ozogamicin, glembatumomab vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMAADC, RG-7450, RG-7458, RG-7593, RG-7596, RG-7598, RG-7599, RG-7600, RG-7636, ABT-414, IMGN-853, IMGN-529, vorsetuzumab mafodotin, and lorvotuzumab mertansine.

***Combination therapy*:** As used herein, the term "combination therapy" refers to those situations in which a subject is simultaneously exposed to two or more therapeutic regimens (e.g., two or more chemotherapeutic agents). The two or more regimens may be administered simultaneously; such regimens may be administered sequentially (e.g., all "doses" of a first regimen are administered prior to administration of any doses of a second regimen); such agents may be administered in overlapping dosing regimens. "Administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although two or more agents, or active moieties thereof, may be administered together in a combination composition, or even in a combination compound (e.g., as part of a single chemical complex or covalent entity).

***Comprising*:** A composition or method described herein as "comprising" one or more named elements or steps is open-ended, meaning that the named elements or steps are essential, but other elements or steps may be added within the scope of the composition or method. To avoid prolixity, it is also understood that any composition or method described as "comprising" (or which "comprises") one or more named elements or steps also describes the corresponding, more limited composition or method "consisting essentially of" (or which "consists essentially of") the same named elements or steps, meaning that the composition or method includes the named essential elements or steps and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "consists of") the named elements or steps to the exclusion of any other unnamed element or step.

***Determine*:** Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. Determining may involve manipulation of a physical sample. Determining may involve consideration and/or manipulation of data or information, for example utilizing a computer or other processing unit adapted to perform a relevant analysis. Determining may involve receiving relevant information and/or materials from a source. Determining may involve comparing one or more features of a sample or entity to a comparable reference.

***Diagnostic information*:** As used herein, "diagnostic information" or "information for use in diagnosis" is information that is useful in determining whether a patient has a disease, disorder or condition and/or in classifying a disease, disorder or condition into a phenotypic category or any category having significance with regard to prognosis of a disease, disorder or condition, or likely response to treatment (either treatment in general or any particular treatment) of a disease, disorder or condition. Similarly, "diagnosis" refers to providing any type of diagnostic information, including, but not limited to, whether a subject is likely to have or develop a disease, disorder or condition, state, staging or characteristic of a disease, disorder or condition as manifested in the subject, information related to the nature or classification of a tumor, information related to prognosis and/or information useful in selecting an appropriate treatment. Selection of treatment may include the choice of a particular therapeutic agent or other treatment modality such as surgery, radiation, etc., a choice about whether to withhold or deliver therapy, a choice relating to dosing regimen (e.g., frequency or level of one or more doses of a particular therapeutic agent or combination of therapeutic agents), etc.

***Gene*:** As used herein, the term "gene" refers to a DNA sequence in a chromosome that codes for a product (e.g., an RNA product and/or a polypeptide product). A gene may include coding sequence (i.e., sequence that encodes a particular product); a gene may include non-coding sequence. A gene may include both coding (e.g., exonic) and non-coding (e.g., intronic) sequences. A gene may include one or more regulatory elements that, for example, may control or impact one or more aspects of gene expression (e.g., cell-type-specific expression, inducible expression, etc.).

***Gene product or expression product*:** As used herein, the term "gene product" or "expression product" generally refers to an RNA transcribed from the gene (pre-and/or post-processing) or a polypeptide (pre- and/or post-modification) encoded by an RNA transcribed from the gene.

***Genome*:** As used herein, the term "genome" refers to the total genetic information carried by an individual organism or cell, represented by the complete DNA sequences of its chromosomes.

***Marker*:** A marker, as used herein, refers to an entity or moiety whose presence or level is a characteristic of a particular state or event. Presence or level of a particular marker may be characteristic of presence or stage of a disease, disorder, or condition. To give but one example, the term may refer to a gene expression product that is characteristic of a particular tumor, tumor subclass, stage of tumor, etc. Alternatively, or additionally, a presence or level of a particular marker may correlate with activity (or activity level) of a particular signalling pathway, for example that may be characteristic of a particular class of tumors. The statistical significance of the presence or absence of a marker may vary depending upon the particular marker. Detection of a marker may be highly specific in that it reflects a high probability that the tumor is of a particular subclass. Such specificity may come at the cost of sensitivity (i.e., a negative result may occur even if the tumor is a tumor that would be expected to express the marker). Conversely, markers with a high degree of sensitivity may be less specific that those with lower sensitivity. According to the present disclosure a useful marker need not distinguish tumors of a particular subclass with 100% accuracy.

***Nucleic acid*:** As used herein, in its broadest sense, refers to any compound and/or substance that is or can be incorporated into an oligonucleotide chain. A nucleic acid may be a compound and/or substance that is or can be incorporated into an oligonucleotide chain via a phosphodiester linkage. As will be clear from context, "nucleic acid" may refer to an individual nucleic acid residue (e.g., a nucleotide and/or nucleoside); "nucleic acid" may refer to an oligonucleotide chain comprising individual nucleic acid residues. A "nucleic acid" may be or comprise RNA; a "nucleic acid" may be or comprise DNA. A nucleic acid may be, comprise, or consist of one or more natural nucleic acid residues. A nucleic acid may be, comprise, or consist of one or more nucleic acid analogs. A nucleic acid analog may differ from a nucleic acid in that it does not utilize a phosphodiester backbone. For example, a nucleic acid may be, comprise, or consist of one or more "peptide nucleic acids", which are known in the art and have peptide bonds instead of phosphodiester bonds in the backbone. Alternatively, or additionally, a nucleic acid may have one or more phosphorothioate and/or 5'-N-phosphoramidite linkages rather than phosphodiester bonds. A nucleic acid may be, comprise, or consist of one or more natural nucleosides (e.g., adenosine, thymidine, guanosine, cytidine, uridine, deoxyadenosine, deoxythymidine, deoxy guanosine, and deoxycytidine). A nucleic acid may be, comprise, or consist of one or more nucleoside analogs (e.g., 2-aminoadenosine, 2-thiothymidine, inosine, pyrrolo-pyrimidine, 3 - methyl adenosine, 5-methylcytidine, C-5 propynyl-cytidine, C-5 propynyl-uridine, 2-aminoadenosine, C5-bromouridine, CS-fluorouridine, CS-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof). A nucleic acid may comprise one or more modified sugars (e.g., 2'-fluororibose, ribose, 2'-deoxyribose, arabinose, and hexose) as compared with those in natural nucleic acids. A nucleic acid may have a nucleotide sequence that encodes a functional gene product such as an RNA or protein. A nucleic acid may include one or more introns. Nucleic acids may be prepared by one or more of isolation from a natural source, enzymatic synthesis by polymerization based on a complementary template (in vivo or in vitro), reproduction in a recombinant cell or system, and chemical synthesis. A nucleic acid may be at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. A nucleic acid may be partly or wholly single stranded; a nucleic acid may be partly or wholly double stranded. A nucleic acid may have a nucleotide sequence comprising at least one element that encodes, or is the complement of a sequence that encodes, a polypeptide. A nucleic acid may have enzymatic activity.

***Patient*:** As used herein, the term "patient" refers to any organism to which a provided composition is or may be administered, e.g., for experimental, diagnostic, prophylactic, cosmetic, and/or therapeutic purposes. Typical patients include animals (e.g., mammals such as mice, rats, rabbits, non-human primates, and/or humans). A patient may be a human. A patient may be suffering from or susceptible to one or more disorders or conditions. A patient may display one or more symptoms of a disorder or condition. A patient may have been diagnosed with one or more disorders or conditions. The disorder or condition may be or include cancer, or presence of one or more tumors. The patient may be receiving or have received certain therapy to diagnose and/or to treat a disease, disorder, or condition.

***Prevent or prevention*:** as used herein when used in connection with the occurrence of a disease, disorder, and/or condition, refers to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Prognostic and predictive information*:** As used herein, the terms "prognostic information" and "predictive information" are used to refer to any information that may be used to indicate any aspect of the course of a disease or condition either in the absence or presence of treatment. Such information may include, but is not limited to, the average life expectancy of a patient, the likelihood that a patient will survive for a given amount of time (e.g., 6 months, 1 year, 5 years, etc.), the likelihood that a patient will be cured of a disease, the likelihood that a patient's disease will respond to a particular therapy (wherein response may be defined in any of a variety of ways). Prognostic and predictive information are included within the broad category of diagnostic information.

***Reference*:** As used herein describes a standard or control relative to which a comparison is performed. For example, an agent, animal, individual, population, sample, sequence or value of interest may be compared with a reference or control agent, animal, individual, population, sample, sequence or value. A reference or control may be tested and/or determined substantially simultaneously with the testing or determination of interest. A reference or control may be a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Response*:** As used herein, a response to treatment may refer to any beneficial alteration in a subject's condition that occurs as a result of or correlates with treatment. Such alteration may include stabilization of the condition (e.g., prevention of deterioration that would have taken place in the absence of the treatment), amelioration of symptoms of the condition, and/or improvement in the prospects for cure of the condition, etc. It may refer to a subject's response or to a tumor's response. Tumor or subject response may be measured according to a wide variety of criteria, including clinical criteria and objective criteria. Techniques for assessing response include, but are not limited to, clinical examination, positron emission tomatography, chest X-ray CT scan, MRI, ultrasound, endoscopy, laparoscopy, presence or level of tumor markers in a sample obtained from a subject, cytology, and/or histology. Many of these techniques attempt to determine the size of a tumor or otherwise determine the total tumor burden. Methods and guidelines for assessing response to treatment are discussed in Therasse et. al., "New guidelines to evaluate the response to treatment in solid tumors", European Organization for Research and Treatment of Cancer, National Cancer Institute of the United States, National Cancer Institute of Canada, J. Natl. Cancer Inst., 2000, 92(3):205-216. The exact response criteria can be selected in any appropriate manner, provided that when comparing groups of tumors and/or patients, the groups to be compared are assessed based on the same or comparable criteria for determining response rate. One of ordinary skill in the art will be able to select appropriate criteria.

***Risk*:** as will be understood from context, "risk" of a disease, disorder, and/or condition refers to a likelihood that a particular individual will develop the disease, disorder, and/or condition. Risk may be expressed as a percentage. Risk may be from 0, 1, 2, 3, 4, 5, 6, 7, 8, 9, 10, 20, 30, 40, 50, 60, 70, 80, 90 up to 100%. Risk may be expressed as a risk relative to a risk associated with a reference sample or group of reference samples. A reference sample or group of reference samples may have a known risk of a disease, disorder, condition and/or event. A reference sample or group of reference samples may be from individuals comparable to a particular individual. Relative risk may be 0,1, 2, 3, 4, 5, 6, 7, 8, 9, 10, or more.

***Sample*:** As used herein, the term "sample" typically refers to an aliquot of material obtained or derived from a source of interest, as described herein. A source of interest may be a biological or environmental source. A source of interest may be or comprise a cell or an organism, such as a microbe, a plant, or an animal (e.g., a human). A source of interest may be or comprise biological tissue or fluid. A biological tissue or fluid may be or comprise amniotic fluid, aqueous humor, ascites, bile, bone marrow, blood, breast milk, cerebrospinal fluid, cerumen, chyle, chime, ejaculate, endolymph, exudate, feces, gastric acid, gastric juice, lymph, mucus, pericardial fluid, perilymph, peritoneal fluid, pleural fluid, pus, rheum, saliva, sebum, semen, serum, smegma, sputum, synovial fluid, sweat, tears, urine, vaginal secreations, vitreous humour, vomit, and/or combinations or component(s) thereof. A biological fluid may be or comprise an intracellular fluid, an extracellular fluid, an intravascular fluid (blood plasma), an interstitial fluid, a lymphatic fluid, and/or a transcellular fluid. A biological fluid may be or comprise a plant exudate. A biological tissue or sample may be obtained, for example, by aspirate, biopsy (e.g., fine needle or tissue biopsy), swab (e.g., oral, nasal, skin, or vaginal swab), scraping, surgery, washing or lavage (e.g., brocheoalvealar, ductal, nasal, ocular, oral, uterine, vaginal, or other washing or lavage). A biological sample may be or comprise cells obtained from an individual. A sample may be a "primary sample" obtained directly from a source of interest by any appropriate means. As will be clear from context, the term "sample" may refer to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to one or more techniques such as amplification or reverse transcription of nucleic acid, isolation and/or purification of certain components, etc.

***Stage of cancer*:** As used herein, the term "stage of cancer" refers to a qualitative or quantitative assessment of the level of advancement of a cancer. Criteria used to determine the stage of a cancer may include, but are not limited to, one or more of where the cancer is located in a body, tumor size, whether the cancer has spread to lymph nodes, whether the cancer has spread to one or more different parts of the body, etc. Cancer may be staged using the so-called TNM System, according to which T refers to the size and extent of the main tumor, usually called the primary tumor; N refers to the number of nearby lymph nodes that have cancer; and M refers to whether the cancer has metastasized. A cancer may be referred to as Stage 0 (abnormal cells are present but have not spread to nearby tissue, also called carcinoma in situ, or CIS; CIS is not cancer, but it may become cancer), Stage I-III (cancer is present; the higher the number, the larger the tumor and the more it has spread into nearby tissues), or Stage IV (the cancer has spread to distant parts of the body.A cancer may be assigned to a stage selected from the group consisting of: in situ (abnormal cells are present but have not spread to nearby tissue); localized (cancer is limited to the place where it started, with no sign that it has spread); regional (cancer has spread to nearby lymph nodes, tissues, or organs): distant (cancer has spread to distant parts of the body); and unknown (there is not enough information to figure out the stage).

***Subject*:** As used herein, the term "subject" refers an organism, typically a mammal (e.g., a human, in some embodiments including prenatal human forms). A subject may be suffering from a relevant disease, disorder or condition. A subject may be susceptible to a disease, disorder, or condition. A subject may display one or more symptoms or characteristics of a disease, disorder or condition. A subject may not display any symptom or characteristic of a disease, disorder, or condition. A subject may be someone with one or more features characteristic of susceptibility to or risk of a disease, disorder, or condition. A subject may be a patient. A subject may be an individual to whom diagnosis and/or therapy is and/or has been administered.

***Therapeutic agent*:** As used herein, the phrase "therapeutic agent" refers to an agent that, when administered to a subject, has a therapeutic effect and/or elicits a desired biological and/or pharmacological effect. A therapeutic agent may be any substance that can be used to alleviate, ameliorate, relieve, inhibit, prevent, delay onset of, reduce severity of, and/or reduce incidence of one or more symptoms or features of a disease, disorder, and/or condition.

***Therapeutic regimen*:** A "therapeutic regimen", as that term is used herein, refers to a dosing regimen whose administration across a relevant population may be correlated with a desired or beneficial therapeutic outcome.

***Treatment*:** As used herein, the term "treatment" (also "treat" or "treating") refers to administration of a therapy that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder and/or condition and/or of a subject who exhibits only early signs of the disease, disorder, and/or condition. Such treatment may be of a subject who exhibits one or more established signs of the relevant disease, disorder and/or condition. Treatment may be of a subject who has been diagnosed as suffering from the relevant disease, disorder, and/or condition. Treatment may be of a subject known to have one or more susceptibility factors that are statistically correlated with increased risk of development of the relevant disease, disorder, and/or condition. Thus, treatment may be prophylactic; treatment may be therapeutic.

***Tumor*:** As used herein, the term "tumor" refers to an abnormal growth of cells or tissue. A tumor may comprise cells that are precancerous (e.g., benign), malignant, pre-metastatic, metastatic, and/or non-metastatic. A tumor may be associated with, or is a manifestation of, a cancer. A tumor may be a disperse tumor or a liquid tumor. A tumor may be a solid tumor.

***Variant*:** As used herein in the context of molecules, e.g., nucleic acids, proteins, or small molecules, the term "variant" refers to a molecule that shows significant structural identity with a reference molecule but differs structurally from the reference molecule, e.g., in the presence or absence or in the level of one or more chemical moieties as compared to the reference entity. A variant may also differ functionally from its reference molecule. In general, whether a particular molecule is properly considered to be a "variant" of a reference molecule is based on its degree of structural identity with the reference molecule. As will be appreciated by those skilled in the art, any biological or chemical reference molecule has certain characteristic structural elements. A variant, by definition, is a distinct molecule that shares one or more such characteristic structural elements but differs in at least one aspect from the reference molecule. To give but a few examples, a polypeptide may have a characteristic sequence element comprised of a plurality of amino acids having designated positions relative to one another in linear or three-dimensional space and/or contributing to a particular structural motif and/or biological function; a nucleic acid may have a characteristic sequence element comprised of a plurality of nucleotide residues having designated positions relative to one another in linear or three-dimensional space. A variant polypeptide or nucleic acid may differ from a reference polypeptide or nucleic acid as a result of one or more differences in amino acid or nucleotide sequence and/or one or more differences in chemical moieties (e.g., carbohydrates, lipids, phosphate groups) that are covalently components of the polypeptide or nucleic acid (e.g., that are attached to the polypeptide or nucleic acid backbone). A variant polypeptide or nucleic acid may show an overall sequence identity with a reference polypeptide or nucleic acid that is at least 85%, 86%, 87%, 88%, 89%, 90%, 91%, 92%, 93%, 94%, 95%, 96%, 97%, or 99%. A variant polypeptide or nucleic acid may not share at least one characteristic sequence element with a reference polypeptide or nucleic acid. A reference polypeptide or nucleic acid may have one or more biological activities. A variant polypeptide or nucleic acid may share one or more of the biological activities of the reference polypeptide or nucleic acid. A variant polypeptide or nucleic acid may lack one or more of the biological activities of the reference polypeptide or nucleic acid. A variant polypeptide or nucleic acid may show a reduced level of one or more biological activities as compared to the reference polypeptide or nucleic acid. A polypeptide or nucleic acid of interest may be considered to be a "variant" of a reference polypeptide or nucleic acid if it has an amino acid or nucleotide sequence that is identical to that of the reference but for a small number of sequence alterations at particular positions. Typically, fewer than about 20%, about 15%, about 10%, about 9%, about 8%, about 7%, about 6%, about 5%, about 4%, about 3%, or about 2% of the residues in a variant are substituted, inserted, or deleted, as compared to the reference. A variant polypeptide or nucleic acid may comprise about 10, about 9, about 8, about 7, about 6, about 5, about 4, about 3, about 2, or about 1 substituted residues as compared to a reference. Often, a variant polypeptide or nucleic acid may comprise a very small number (e.g., fewer than about 5, about 4, about 3, about 2, or about 1) number of substituted, inserted, or deleted, functional residues (i.e., residues that participate in a particular biological activity) relative to the reference. A variant polypeptide or nucleic acid may comprise not more than about 5, about 4, about 3, about 2, or about 1 addition or deletion, and, , may comprise no additions or deletions, as compared to the reference. A variant polypeptide or nucleic acid may comprise fewer than about 25, about 20, about 19, about 18, about 17, about 16, about 15, about 14, about 13, about 10, about 9, about 8, about 7, about 6, and commonly fewer than about 5, about 4, about 3, or about 2 additions or deletions as compared to the reference. A reference polypeptide or nucleic acid may be one found in nature. A reference polypeptide or nucleic acid may be a human polypeptide or nucleic acid.

### Detailed Description of Certain Embodiments

The present invention is defined in the appended claims.

### Cytosine Modifications

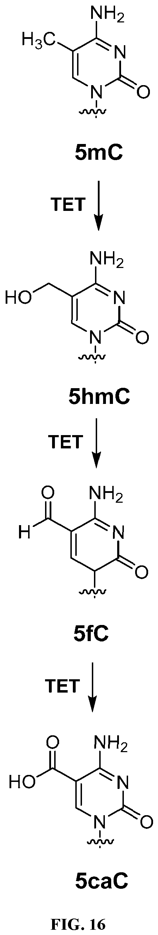

The primary DNA sequence of the four-letter alphabet G, C, A and T forms the genetic information of life on earth. Chemical modifications of DNA bases do not change the underlying sequence, but instead carry an extra layer of information. The first discovered 5-methylcytosine (5mC) is the most studied modified base, and it plays crucial roles in a broad range of biological processes from gene regulation to normal development (See, for example, Li et al. "DNA methylation in mammals" Cold Spring Harb. Perspect. Bio., 6, 2014) and is regarded as the fifth base. 5-hydroxymethylcytosine (5hmC) is converted from SmC by the ten-eleven translocation (TET) family of dioxygenase (See, for example, Tahiliani et al. "Conversion of 5-methylcytosine to 5-hydroxymethylcytosine in mammalian DNA by MLL partner TET1" Science, 324, 2009); it is enriched in neuronal cells (See, for example, Kriaucionis et al. "The nuclear base 5-hydroxymethylcytosine is present in Purkinje neurons and the brain" Science, 324, 2009) and regarded as the sixth base. Further successive TET oxidation results in 5-formylcytosine (5fC) and 5-carboxylcytosine (ScaC) (Figure 16) (See, for example, Ito et al. "Tet proteins can convert 5-methylcytosine to 5-formylcytosine and 5-carboxylcytosine" Science, 333, 2011 and He et al. "Tet-mediated formation of 5-carboxycytosine and its excision by TDG in mammalian DNA" Science, 333, 2011, which exist at much lower abundances in the mammalian genome and are regarded as intermediates in the thymine DNA glycosylase (TDG)-base excision repair (BER) active demethylation pathway (See, for example, He et al. "Tet-mediated formation of 5-carboxycytosine and its excision by TDG in mammalian DNA" Science, 333, 2011). Emerging evidence indicates the stability of these DNA demethylation intermediates (See, for example, Bachman et al. "5-Formylcytosine can be a stable DNA modification in mammals" Nat. Chem. Biol., 11, 2015) as well as potential functional role (See, for example, Kellinger et al. "5-formylcytosine and 5-carboxylcytosine reduce the rate and substrate specificity of RNA polymerase II transcription" Nat. Struct. Mol. Biol., 19, 2012).

5-Methylcytosine (SmC) and 5-hydroxymethylcytosine (5hmC) are the two major epigenetic marks found in the mammalian genome. ShmC is generated from SmC by the ten-eleven translocation (TET) family dioxygenases. Tet can further oxidize ShmC to 5- formylcytosine (5fC) and 5-carboxylcytosine (ScaC), which exists in much lower abundance in the mammalian genome compared to SmC and ShmC (10-fold to 100-fold lower than that of ShmC). Together, SmC and ShmC play crucial roles in a broad range of biological processes from gene regulation to normal development. Aberrant DNA methylation and hydroxymethylation have been associated with various diseases and are well-accepted hallmarks of cancer. Therefore, the determination of SmC and ShmC in DNA sequence is not only important for basic research, but also is valuable for clinical applications, including diagnosis and therapy.

SfC and ScaC are the two final oxidized derivatives of SmC and can be converted to unmodified cytosine by Thymine DNA glycosylase (TDG) in base excision repair pathway. Therefore, SfC and ScaC are two important key intermediates in the active demethylation process, which plays important role in embryonic development. SfC and ScaC are found in these contexts and may serve as indicator of nearly complete SmC demethylation. SfC and ScaC may also play additional functions such as bind specific proteins and affect the rate and specificity of RNA polymerase II. SfC has also been detected in human and animal tRNA, and may play a role in certain mitochondrial diseases (See, for example, Dietzsch et al., "Chemoselective labeling and site-specific mapping of 5-formylcytosine as a cellular nucleic acid modification", FEBS Letters, 592:12, 2018; and Moriya et al., "A Novel Modified Nucleoside Found at the First Position of the Anticodon of Methionine tRNA from Bovine Liver Mitochondria", Biochemistry, 33, 1994).

SmC is also a post-transcriptional RNA modification that has been identified in both stable and highly abundant tRNAs and rRNAs, and in mRNAs. In addition, SmC has been detected in snRNA (small nuclear RNA), miRNA (microRNA), lncRNA (long noncoding RNA) and eRNA (enhancer RNA). However, there appears to be differences in the occurrence of SmC in specific RNA types in different organisms. For example, SmC appears not to be present in tRNA and mRNA from bacteria, while it has been found in tRNA and mRNA in eukaryotes and archaea.

ShmC has also been detected in RNA. For example, mRNA from Drosophila and mouse has been found to contain 5hmC. The same family of enzymes that oxidize SmC in DNA was reported to catalyze the formation of ShmC in mammalian total RNA. In flies, a transcriptome wide study using methylation RNA immunoprecipitation sequencing (MeRIPseq) with ShmC antibodies, detected the presence of ShmC in many mRNA coding sequences, with particularly high levels in the brain. It was also reported that active translation is associated with high ShmC levels in RNA, and flies lacking the TET enzyme responsible for ShmC deposition in RNA have impaired brain development.

### Source Nucleic Acid

Technologies provided herein are useful for analyzing cytosine modifications in any of a variety of source nucleic acids. In principle, a source nucleic acid can be any nucleic acid present in a source of interest (e.g., in a biological or environmental sample), or generated by the hand of man, e.g., by chemical synthesis, template-directed synthesis, and/or modification.

In some embodiments, a source nucleic acid is or comprises DNA. In some embodiments, a source nucleic acid is or comprises RNA. In some embodiments, a source nucleic acid is or comprises genomic DNA. In some embodiments, a source nucleic acid is or comprises RNA (e.g., mRNA, tRNA, ncRNA).

In some embodiments, a source nucleic acid may be or comprise a population of nucleic acid molecules.

In some embodiments, one or more nucleic acid molecules in a source nucleic acid may include one or more cytosine modifications (e.g., SmC, ShmC, 5fC, and/or Scac). In some embodiments, a cytosine modification may be present in a nucleic acid as obtained (e.g., isolated) from a source of interest (e.g., a biological or environmental source). In some embodiments, a cytosine modification may be introduced by the hand of man (e.g., by manipulating one or more features of a cell or system, or by performing a chemical reaction in vitro).

In some embodiments, one or more nucleic acid molecules in a source nucleic acid may include one or more tagged or blocked residues (e.g., cytosine or modified cytosine residues). For example, in some embodiments, a source nucleic acid may include one or more tagged or blocked 5mC, ShmC, SfC, and/or ScaC residues, as described herein.

In some embodiments, analysis as described herein is applied to one or more particular sequences within a source nucleic acid; e.g., cytosine modification is assessed for one or more particular target nucleic acid sequences that are present in nucleic acids from a source of interest.

In some embodiments, a source of interest (e.g., where a source nucleic acid may be found and/or from which a source nucleic acid may be obtained) may be or comprise a cell, tissue, or organism from a Kingdom such as the Monera (bacteria), Protista, Fungi, Plantae, or Animalia Kingdoms. In some embodiments, a source nucleic acid may be obtained from a patient or subject (e.g., may be in a biological sample), from an environmental sample, or from an organism of interest, among other things.

In embodiments, a source nucleic acid is extracted or otherwise obtained from a cell or collection of cells, a body fluid, a tissue sample, an organ, and an organelle. In some embodiments, a source nucleic acid is from one or more tumor cells. In some embodiments, a source nucleic acid is from one or more embryonic stem cells. In some embodiments, a source nucleic acid is extracted or derived from blood and/or plasma (e.g., human blood and/or plasma).

In some embodiments, a source nucleic acid may be subjected to one or more manipulation and/or processing steps (e.g., fragmentation or cleavage, tagging, isolation [e.g., which may involve hybridization, precipitation, purification, affinity pull-don, tagging, blocking of one or more residues or moieties, etc) prior to analysis as described herein.

In some embodiments, a nucleic acid subjected to analysis as described herein may contain (e.g., may have been manipulated/modified to contain) one or more blocked cytosine groups (e.g. a blocked 5mC, ShmC, 5fC, and/or ScaC) prior to further oxidation and//or reduction reaction as described herein.

In some embodiments, a source nucleic acid may be or comprise a synthetic nucleic acid (e.g., a sequence created partially or entirely by man, either in vivo or ex vivo). In some embodiments, a source nucleic acid can be a nucleic acid present in a source of interest (e.g., in a biological or environmental sample), or generated by the hand of man, e.g., by chemical synthesis, template-directed synthesis, and/or modification.

In some embodiments, a source nucleic acid is or comprises one or more target nucleic acid. In some embodiments, a target nucleic acid can be a single nucleic acid molecule in a sample; in some embodiments, a target nucleic acid may be a plurality of nucleic acid molecules, or even may be the entire population of nucleic acid molecules in a sample. In some embodiments, a target nucleic acid can be a native nucleic acid from a source (e.g., cells, tissue samples, etc.); in some embodiments, a target nucleic acid may have been manipulated (e.g., pre-converted into a high-throughput sequencing-ready form, for example by fragmentation, repair and ligation with adaptors for sequencing).

In some embodiments, a target nucleic acid may be one that is or comprises a particular nucleic acid sequence of interest.

In some embodiments, the present disclosure involves preparing or obtaining a plurality of target nucleic acids that can be analyzed (e.g., individually) as described herein. In some embodiments, such plurality may include nucleic acid(s) with one or more common features or characteristics (e.g., presence of one or more presence of one or more particular sequence elements, preparation from a common source - e.g., a common organism, tissue, cell type, etc., including for example preparation from a particular tumor or other diseased tissue): in some embodiments, such a plurality may be referred to as a "collection" or "library"; the term "library" being particularly used when nucleic acids share one or more common features or characteristics.

In some embodiments, provided technologies (e.g., provided oxidation and/or reduction technologies) are utilized together with and/or otherwise in the context of nucleic acid sequencing technologies - e.g., that involve determining a nucleotide sequence (and/or location and/or type of one or more cytosine modifications therein) of a single nucleic acid molecule, and/or of a plurality of individual nucleic acid molecules and/or of a group of nucleic acid molecules. In some embodiments, technologies such as high-throughput and/or next generation sequencing are utilized.

### Analyzing Cytosine Modifications

Detection and/or analysis of cytosine modifications have presented intriguing challenges for scientists. Although various methods have been developed for sequencing cytosine modifications, it is still challenging to generate specific and quantitative sequence information for individual modification at base-resolution. Traditionally, bisulfite sequencing (BS) has been the gold standard for base-resolution and quantitative analysis of SmC and ShmC (See, for example, Darst et al. "Bisulfite sequencing of DNA" Curr. Protoc. Mol. Biol., Chapter 7, Unit 7, 2010). Modified BS has also been developed for specific sequencing of 5mC (oxidative bisulfite sequencing, oxBS-seq) (See, for example, Booth et al. "Quantitative sequencing of 5-methylcytosine and 5-hydroxymethylcytosine at single-base resolution" Science, 336, 2012) or ShmC (TET-assisted bisulfite sequencing, TAB-seq) (See, for example, Yu et al. "Base-resolution analysis of 5-hydroxymethylcytosine in the mammalian genome" Cell, 149, 2012). These methods, however, all involve harsh bisulfite treatment, which degrades up to 99% of the DNA (See, for example, Tanaka et al. "Degradation of DNA by bisulfite treatment" Bioorg. Med. Chem., 17, 2007), and reduces sequence complexity by converting unmodified cytosine (~95% of the total cytosine in the human genome) to thymine (T).

Recently, bisulfite-free quantitative base-resolution methods have emerged and showed significant advantages over BS (See, for example, Zhao et al. "Mapping the epigenetic modifications of DNA and RNA" Protein Cell, 2020). Among them, APOBEC-coupled epigenetic sequencing (ACE-seq, which detects ShmC) and Enzymatic Methyl-seq (EM-seq, which detects SmC+ShmC) use an enzymatic deamination step to replace the bisulfite deamination step (See, for example, Schutsky et al. "Nondestructive, base-resolution sequencing of 5-hydroxymethylcytosine using a DNA deaminase" Nat. Biotechnol., 36, 2018; and Vaisvila et al. "Detection of DNA Methylation at Single Base Resolution from Picograms of DNA" bioRxiv, 2020). While these methods solve the DNA damage issue, they still suffer from the indirect detection issue of BS by converting unmodified cytosine to T. Recently, a TET assisted pyridine borane sequencing (TAPS) method based on a novel pyridine borane reductive decarboxylation and deamination chemistry was developed (See, for example, Liu et al. "Bisulfite-free direct detection of 5-methylcytosine and 5-hydroxymethylcytosine at base resolution" Nat. Biotechnol., 37, 2019; and Liu et al. "Accurate targeted long-read DNA methylation and hydroxymethylation sequencing with TAPS" Genome Bio., 21, 2020). In TAPS, SmC and ShmC are oxidized by TET proteins to ScaC and reduced to dihydrouracil (DHU) by pyridine borane. DHU is then amplified and sequenced as T during sequencing. TAPS is nondestructive and detects SmC+ShmC directly, and it shows improved sequence quality, mapping rate and coverage compared to BS.

SmC and ShmC provide distinct and antagonistic epigenetic information: SmC usually marks repressed genes and ShmC generally marks expressed genes (See, for example, Mellen et al. "MeCP2 binds to ShmC enriched within active genes and accessible chromatin in the nervous system" Cell, 151, 2012). To elucidate the interplay between SmC and ShmC in various biological processes, it is necessary to distinguish the two modifications. Currently accepted methods to distinguish SmC and ShmC require two or more assays (e.g. BS and oxBS-seq, BS and TAB-seq, or EM-Seq and ACE-seq) be performed and a subtraction between the two assays is usually required to obtain both SmC and ShmC information (e.g. BS minus oxBS-seq to get ShmC, BS minus TAB-seq to get SmC, or EM-Seq minus ACE-seq to get 5mC). However, subtraction may introduce negative values because of random sampling or systematic error in each experiment and suffer from accumulation of noise from multiple assays, which increases the need for higher sequencing depth (See, for example, He et al. "DeepH&M: Estimating single-CpG hydroxymethylation and methylation levels from enrichment and restriction enzyme sequencing methods" Science Advances, 6, 2020,) as well as more effort to perform read filtering and apply statistical tests (See, for example, Qu et al. "MLML: consistent simultaneous estimates of DNA methylation and hydroxymethylation" Bioinformatics, 29, 2013). A subtraction-free approach where two assays (e.g. oxBS-seq and TAB-seq) can read out the true SmC and true ShmC information directly is desirable. Therefore, there is an unmet need for easily accessible, subtraction-free methods that can provide base-resolution sequencing of cytosine modifications with high efficiency and accuracy.

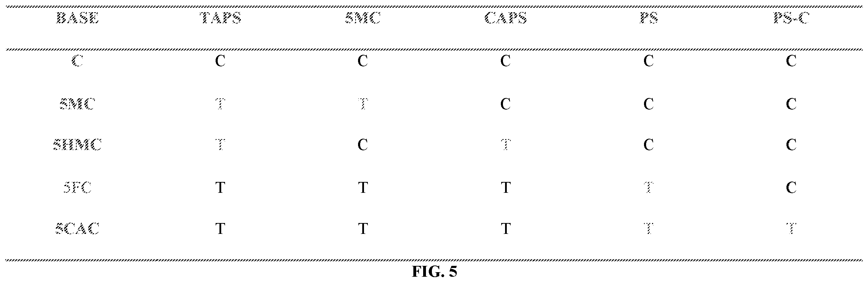

The present disclosure demonstrates, among other things, versatility of borane reduction chemistry for direct and quantitative sequencing of all four individual cytosine modification in mouse embryonic stem cells (mESCs), for example by presenting TAPS with βGT blocking (TAPSβ) and chemical-assisted pyridine borane sequencing (CAPS) for whole-genome subtraction-free and specific sequencing of true SmC and true ShmC, respectively; and pic/pyridine borane sequencing (PS) for whole-genome sequencing of 5fC and ScaC (Fig. 5).

The present disclosure provides, among other things, bisulfite-free, base-resolution methods for detecting (e.g., identifying) cytosine modifications in a nucleic acid (e.g., in whole genomic DNA). Technologies described herein include improvements to those described in WO/2019/136413, and which describes methods including a bisulfite-free, base-resolution method for detecting SmC and ShmC in a sequence that has been named "TAPS". TAPS consists of mild enzymatic and chemical reactions to detect SmC and ShmC directly and quantitatively at base-resolution without affecting unmodified cytosine. In some embodiments, TAPS may be performed on a nucleic acid sample that has been reacted with a glucosyltransferase (e.g., a β-glucosyltransferase), such that ShmC residues are modified with a carbohydrate (e.g., glucose) moiety; such embodiments are often referred to as "TAPSβ".

Among other things, the present disclosure confirms that TAPS, TAPSβ and PS efficiently identify cytosine modifications in genomic DNA. Alternatively, or additionally, the present disclosure also provides improved technologies for detecting ShmC at base resolution, for example through the use of improved CAPS reagents and methods (which may, in some embodiments, be used alone or alternatively together with one or more other technologies, including for example TAPS, TAPSβ and/or PS). Thus, the present disclosure provides, among other things, improved technologies for mapping of ShmCand can overcome disadvantages of previous methods, specifically including those that involve bisulfite treatment.

Provided technologies permit assessment of cytosine modifications in nucleic acid preparations, such as preparations of source nucleic acids (themselves and/or manipulated and/or modified embodiments thereof). As noted above, in some embodiments, a source nucleic acid is a nucleic acid present in and/or obtained from a source of interest (e.g., a cell, tissue, organism, or sample of interest).

In many embodiments, assessment technologies described herein include an oxidation step and/or a reduction step, performed in vitro. In some embodiments, such oxidation step or reduction step may be performed on source nucleic acid as isolated or obtained from the source. In some embodiments, one or more processing steps may be or have been performed on a source nucleic acid prior to performance of a particular reaction as described herein. Alternatively, or additionally, in some embodiments, one or more additional processes (e.g., purification/isolation) and/or modification steps may be performed between provided reactions, or after one or more provided reactions. Thus, in some embodiments, reactions as described herein may be applied to nucleic acids that have already been subjected to (and, in some embodiments, modified by) one or more processing steps (i.e., that are processed nucleic acids).

In some embodiments, processing steps may include one or more of isolation/purification, cleavage, digestion, and chemical modification, among other things.

To give but a few examples, in some embodiments, particular residue(s) in a source nucleic acid may be modified (e.g., tagged or blocked) prior to performance of a reaction (e.g., an oxidation or reduction reaction) as described herein (for example so that such residue is excluding from reacting in the relevant reaction).

In some embodiments, technologies and methods provided herein may be used for analysis of disease prognosis and/or likely response to treatment (e.g. for one or more different cancers). In some embodiments, technologies and methods provided herein can be used for diagnosis and/or risk assessment for certain diseases (e.g. one or more different types of cancer). In some embodiments, technologies and methods provided herein may be used to inform selection of a particular therapy (e.g., a cancer therapy such as chemotherapy, immunotherapy, etc.) for treatment of a disease (e.g. one of more different types of cancer).

In some embodiments, information gained through analysis of cytosine modifications (e.g., sequence information and/or quantification) can be used for diagnosis and/or risk assessment of disease. In some embodiments, information gained through analysis of cytosine modifications (e.g., sequence information and/or quantification) can be used for diagnosis and/or risk assessment for certain cancers.

In some embodiments, provided technologies utilize one or more oxidation and/or reduction reactions as described herein and do not utilize an affinity purification step (e.g., a pulldown). In some embodiments, provided technologies may utilize an affinity purification step, but not immediately before an oxidation step as described herein. In some embodiments, provided technologies may utilize an affinity purification step, but not immediately after an oxidation step as described herein. In some embodiments, provided technologies may utilize an affinity purification step, but not immediately before a reduction step as described herein. In some embodiments, provided technologies may utilize an affinity purification step, but not immediately after a reduction step as described herein. For example, in some embodiments, provided technologies utilize one or more oxidation and/or reduction reaction as described herein and do not utilize an enrichment step for 5hmC (e.g., an antibody- and/or tag-based pulldown).

In some embodiments, provided technologies do not utilize bisulfite treatment following one or more oxidation steps. In some embodiments, provided technologies do not utilize bisulfite treatment in any step.

In some embodiments, nucleic acid preparation (e.g., isolation and/or manipulation of a source nucleic acid) comprises one or more oxidation steps, one or more reduction steps, and/or one or more sequencing steps. In some embodiments, nucleic acid preparation comprises one or more blocking steps. In some embodiments, nucleic acid preparation comprises one or more amplification steps. In some embodiments, nucleic acid preparation comprises one or more purification steps. In some embodiments, nucleic acid preparation does not comprise any purification steps. In some embodiments, nucleic acid preparation comprises one or more enrichment steps. In some embodiments, nucleic acid preparation does not comprise any enrichment steps.

### Oxidation

Certain technologies provided herein are useful for selectively oxidizing one or more cytosine modifications in a nucleic acid (e.g., a source nucleic acid, such as an isolated source nucleic acid, or a processed nucleic acid).

Selective oxidation of cytosine modifications has been reported previously through the use of certain oxidizing reagents, including dioxygenase enzymes (e.g., TET dioxygenases) and metal oxides (e.g., potassium perruthenate, KRuO4). Ten-eleven translocation (TET) dioxygenases are enzymes that can selectively oxidize SmC to ShmC, SfC, and/or ScaC in a series of successive reactions. Certain metal oxides can be employed for selective oxidation of ShmC to 5fC, including, e.g., potassium perruthenate. However, previous metal oxide-based methods often suffer from increased DNA-damaging effects and/or high false positive rates (See, for example, Zeng et al. "Bisulfite-Free, Nanoscale Analysis of 5-Hydroxymethylcytosine at Single Base Resolution" J. Am. Chem. Soc., 140, 2018; WO/2013/017853; and WO/2014/083118).

Various technologies have been developed that employ one or more of these selective oxidation steps in methods analyzing cytosine modifications, including, e.g., Tet-assisted bisulfite sequencing (TAB-Seq) and oxidative bisulfite sequencing (oxBS-Seq) (See, for example, Yu et al. "Base-resolution analysis of 5-hydroxymethylcytosine in the mammalian genome" Cell, 149:6, 2012; and Booth et al. "Quantitative sequencing of 5-methylcytosine and 5-hydroxylmethylcytosine at single-base resolution, Science, 336, 2012). However, many such techniques require the use of bisulfite, which acts upon unmodified cytosines and is known to cause widespread nucleic acid degradation (See, for example, Tanaka et al., "Degradation of DNA by bisulfite treatment", Bioorg. Med. Chem. Lett., 17, 2007).

Previous work by Liu *et al.* has shown that initial TET or perruthenate oxidation reactions can be followed by a relatively mild borane reduction step (e.g., with pyridine borane and/or pic-borane (pic-BH3)) and sequencing step (e.g., TAPS or CAPS, as described herein) to analyze cytosine modifications at base-resolution with minimal nucleic acid damage. In some embodiments, the present disclosure provides an insight that an oxidation step of CAPS can be further optimized to provide certain desired effects (e.g., reduced false positive rate, reduced nucleic acid damage, increased conversion efficiency, among other things), through use of particular methods and reagents as described herein.

A suitable oxidizing agent may be a reagent that performs an oxidation step on a cytosine modification (e.g., SmC, ShmC, 5fC, and/or ScaC) without affecting unmodified cytosine (C). For example, suitable oxidizing agents may include an organic compound, inorganic compound, protein, and/or nucleic acid, among other things, that perform the desired function. A suitable oxidizing reagent may convert a cytosine modification (e.g., SmC or ShmC) to SfC and/or ScaC.

An oxidizing agent may be or include a native or engineered protein. An oxidizing agent may include or comprise a TET enzyme (e.g., TET1, TET2, or TET3). An oxidizing agent may be or comprise a TET enzyme from an animal (e.g., human TET1, human TET2, human TET3, murine TET1, murine TET2, murine TET3, Naegleria TET (NgTET), Coprinopsis cinerea (CcTET)), or a variant thereof. An oxidizing agent may be or comprise a TET enzyme from a mammal, or a variant thereof. An oxidizing agent may be or comprise an engineered TET enzyme. An oxidizing agent may be human TET1 (hTET1), or a variant thereof (e.g., hTET1CD). An oxidizing agent may be mouse TET1 (mTET1), or a variant thereof (e.g., mTET1CD). An oxidizing agent may be human TET2 (hTET2), or a variant thereof.

An oxidizing agent may be or comprise a TET polypeptide that is characterized by presence of one or more characteristic sequence elements found in known TET enzymes and/or overall sequence identity with a reference TET enzyme of interest (e.g., a reference human TET enzyme or a reference mouse TET enzyme; certain exemplary reference TET enzymes are listed below in Table 1.

**Table 1**

| Enzyme | Gene ID | GenBank Protein ID |
|---|---|---|
| Human TET1 (hTET1) | 80312 | NP 085128 |
| Human TET2 (hTET2) | 54790 | NP 001120680 |
| Human TET3 (hTET3) | 200424 | NP_001274420.1 |
| Mouse TET1 (mTET1) | 52463 | NP_001240786.1 |
| Mouse TET2 (mTET2) | 214133 | NP_001035490.2 |
| Mouse TET3 (mTET3) | 194388 | NP_001334242.1 |

.

A TET polypeptide may be utilized in a TAPS or TAPSβ reaction.

An oxidizing agent may be or comprise a metal oxide compound and/or complex. An oxidizing agent may be or comprise an inorganic metal oxide compound and/or complex. An oxidizing agent is a metal (VI) oxo complex. An oxidizing agent may be or comprise a metal (VII) oxo complex. In some embodiments, an oxidizing agent may be or comprise a ruthenate compound and/or complex. In some embodiments, an oxidizing agent may be or comprise a potassium ruthenate compound and/or complex.

In some embodiments, a metal oxide oxidizing agent, and in particular a metal (VI) oxo complex, and further in particular a ruthenate compound, is utilized in a CAPS reaction.

In some embodiments, an oxidation reaction may be performed at a temperature within a range of 4°C to 40°C. In some embodiments, an oxidation reaction may be performed at a temperature above 4°C. In some embodiments, an oxidation reaction may be performed at a temperature of 37°C.

### Reduction

Certain technologies provided herein are useful for selectively reducing one or more cytosine modifications in a nucleic acid (e.g., a source nucleic acid, such as an isolated source nucleic acid, or a processed nucleic acid).

As previously described in Liu *et al.,* bisulfite sequencing was previously commonly accepted as the standard method for detection of certain cytosine modifications. The development of TAPS and CAPS technologies demonstrated a mild, easily accessible set of reactions that incorporated, among other things, a reducing step to convert native or previously generated SfC and/or ScaC residues to DHU. This reducing step can be used alone, e.g. in PS, or in combination with one or more oxidation, amplification, and/or blocking steps to provide information on the location and/or abundance of cytosine modifications in a nucleic acid. Provided TAPS, TAPSβ, CAPS, and/or PS technologies demonstrate a non-damaging, high-efficiency alternative to bisulfite sequencing for analysis of various cytosine modifications.

In some embodiments, a suitable reducing agent can be a reagent that performs a reducing step on a particular cytosine modification (e.g., 5fC, and/or ScaC) without affecting unmodified cytosine (C) or other cytosine modifications (e.g., Smc and/or ShmC). For example, in some embodiments, suitable reducing agents may include an organic compound, inorganic compound, protein, and/or nucleic acid, among other things, that perform the desired function. A reducing agent converts a cytosine modification (e.g., a SfC and/or ScaC) to dihydrouracil (DHU). In some embodiments, a reducing agent may include or comprise a borane. In some embodiments, a reducing agent is one or more of pyridine borane, 2-picoline borane (pic-BH3), borane, sodium borohydride, sodium cyanoborohydride, and/or sodium triacetoxyborohydride.

In some embodiments, a reducing reaction as described herein (e.g., utilizing a reducing agent as described herein, such as a metal oxide reducing agent, e.g., a metal (VI) oxo complex reducing agent, and particularly a ruthenate compound) is performed, in part or in its entirety, at a pH below about 8; in some embodiments, such pH is below about 7; in some embodiments such pH is below about 6. In some embodiments, a reducing reaction as described herein is performed within a pH range of about 5 to about 6. In some embodiments, a reducing reaction as described herein comprises an alcohol, In some embodiments, a reducing reaction as described herein comprises a salt. In some embodiments, a reducing reaction as described herein comprises a sodium salt. In some embodiments, a reducing reaction as described herein comprises an acid. In some embodiments, a reducing reaction as described herein comprises acetic acid and/or acetate.

In some embodiments (not according to the claimed invention), the present disclosure provides compositions comprising a nucleic acid (e.g. a source nucleic acid) and a metal oxide agent (e.g., a metal (VI) oxo complex, which may be or comprise a ruthenate compound, e.g., potassium ruthenate) and having a pH below about 8; in some embodiments (not according to the claimed invention), such pH is below about 7; in some embodiments (not according to the claimed invention), such pH is below about 6. In some embodiments (not according to the claimed invention), a composition as described herein is within a pH range of about 5 to about 6. In some embodiments (not according to the claimed invention), a composition as described herein comprises an alcohol. In some embodiments (not according to the claimed invention), a composition as described herein comprises a salt. In some embodiments (not according to the claimed invention), a composition as described herein comprises a sodium salt. In some embodiments (not according to the claimed invention), a composition as described herein comprises an acid. In some embodiments (not according to the claimed invention), a composition as described herein comprises acetic acid and/or acetate.

### Amplification

In some embodiments, assessments as described herein may include one or more amplification steps.

In some embodiments, one or more amplification steps may be performed on a nucleic acid (e.g., a source nucleic acid, such as an isolated source nucleic acid, or a processed nucleic acid). In some embodiments, one or more amplification steps may be performed on DNA (e.g., genomic DNA and/or circulating free DNA (cfDNA)). In some embodiments, one or more amplification steps may be performed on RNA (e.g., mRNA, tRNA, and/or ncRNA).

In some embodiments, one or more amplification steps may be performed before and/or after one or more oxidation steps. In some embodiments, one or more amplification steps may be performed before and/or after one or more reduction steps.

In some embodiments, one or more amplification steps may modify the copy number of a nucleic acid (e.g., a source nucleic acid, such as an isolated source nucleic acid or a processed nucleic acid). In some embodiments, one or more amplification steps is performed prior to detecting and/or analyzing the sequence of a source nucleic acid. In some embodiments, one or more amplification steps may comprise one or more of polymerase chain reaction (PCR), primer extension, and/or cloning. In some embodiments, one or more amplification steps may comprise reverse transcription PCR (RT-PCR) using one or more of oligo(dT) primers, random primers, and/or gene specific primers.

In some embodiments, one or more amplification steps may comprise cloning a nucleic acid sequence into a DNA vector by standard techniques. In some embodiments, one or more amplification steps may comprise PCR amplification to generate a library of nucleic acid sequences. In some embodiments, one or more amplification steps may comprise PCR amplification to generate a library of nucleic acid sequence for high-throughput sequencing. In some embodiments, one or more amplification steps may comprise ligation of one or more adaptor sequences (e.g., single-stranded or double-stranded adaptors), followed by PCR.

### Sequencing

In some embodiments, assessments as described herein may include one or more sequencing steps.

In some embodiments, one or more sequencing steps may be performed on a nucleic acid (e.g., a source nucleic acid, such as an isolated source nucleic acid, or a processed nucleic acid). In some embodiments, one or more sequencing steps may be performed on DNA (e.g., genomic DNA and/or circulating free DNA (cfDNA)). In some embodiments, one or more sequencing steps may be performed on RNA (e.g., mRNA, tRNA, and/or ncRNA).

In some embodiments, one or more sequencing steps may be performed before and/or after one or more oxidation steps. In some embodiments, one or more sequencing steps may be performed before and/or after one or more reduction steps. In some embodiments, one or more sequencing steps may be compared relative to a reference nucleic acid (e.g., a source nucleic acid, such as an isolated source nucleic acid, or a processed nucleic acid). In some embodiments, one or more sequencing steps is not preceded by bisulfite treatment. In some embodiments, one or more sequencing steps is not preceded by an amplification step. In some embodiments, one or more sequencing steps is not preceded by an enrichment step. In some embodiments, one or more sequencing steps detects a cytosine modification by identifying a C to T transition in a nucleic acid sequence.

In some embodiments, one or more sequencing steps may comprise a sequencing method known and/or described in the art (e.g., Sanger sequencing, high-throughput sequencing, next generation sequencing (NGS)).

### Enrichment

In some embodiments, assessments as described herein may include one or more enrichment steps.

Previous technologies have been developed for enrichment of nucleic acid sequences comprising one or more cytosine modifications. For example, antibody- and/or biotin-based pulldown methods have been used to enrich DNA sequences comprising ShmC (e.g., ShmC-DIP, hmC-Seal, CMS-seq, ShmC-CATCH, and GLIB-Seq) (See, for example, Zeng et al. "Bisulfite-Free, Nanoscale Analysis of 5-Hydroxymethylcytosine at Single Base Resolution" J. Am. Chem. Soc., 140, 2018; Booth et al., "Chemical methods for decoding cytosine modifications in DNA", Chem. Rev., 115:6, 2015; Plongthongkum, et al., "Advances in the profiling of DNA modifications: cytosine methylation and beyond", Nat. Rev. Genet., 15:10, 2014). A method comprising one or more enrichment steps can increase the abundance of nucleic acids of interest (e.g., a source nucleic acid, such as an isolated source nucleic acid, or a processed nucleic acid); however, it is important to note that such a method does not enable absolute quantitation of cytosine modifications (e.g., SmC, ShmC, 5fC, and/or ScaC).

In some embodiments, one or more enrichment steps may be performed on a nucleic acid (e.g., a source nucleic acid, such as an isolated source nucleic acid, or a processed nucleic acid). In some embodiments, one or more enrichment steps may be performed on DNA (e.g., genomic DNA and/or circulating free DNA (cfDNA)). In some embodiments, one or more enrichment steps may be performed on RNA (e.g., mRNA, tRNA, and/or ncRNA).

In some embodiments, one or more enrichment steps may be performed before and/or after one or more oxidation steps. In some embodiments, one or more enrichment steps may be performed before and/or after one or more reduction steps. In some embodiments, one or more enrichment steps may be preceded by ligation of a nucleic acid adaptor. In some embodiments, one or more enrichment steps may be preceded by reaction of a cytosine modification with a chemical reagent to produce a cytosine modification comprising a chemical moiety of interest (e.g., comprising a handle for biotin conjugation).

In some embodiments, one or more sequencing steps may comprise and/or be performed on a nucleic acid that has been subject to e.g., immediately prior to sequencing) an enrichment method known and/or described in the art (e.g., antibody-based pulldown, biotin-based pulldown, etc.).

Those skilled in the art, reading the present disclosure will appreciate that certain other technologies (e.g., nucleic acid manipulation technologies) may be used together with one or more technologies described herein.

### Kits

In some embodiments (not according to the claimed invention), the present disclosure provides kits that comprise sets of components (e.g., reagents, buffers, and/or reference materials, etc) that, among other things, may be useful as described herein, e.g., for identification of cytosine modifications (e.g., of SmC, ShmC, SfC, ScacC, etc) in a nucleic acid.

In some embodiments (not according to the claimed invention), provided kits may comprise components useful for detection (e.g., identification) of 5mC and/or 5hmC) as described herein. Alternatively, or additionally, in some embodiments (not according to the claimed invention), provided kits may contain components for detection (e.g., identification) of ScaC and/orSfC as described herein.

In some embodiments (not according to the claimed invention), a kit comprises a TET polypeptide as described herein, a borane reducing agent as described herein, and instructions for performing a method; in some particular such embodiments (not according to the claimed invention), the TET polypeptide is TET1 and the borane reducing agent is selected from one or more of the group consisting of pyridine borane, 2-picoline borane (pic-BH3), borane, sodium borohydride, sodium cyano borohydride, and sodium triacetoxyborohydride, or the TET1 polypeptide is NgTetl or murine TETl and the borane reducing agent is pyridine borane and/or pic-BH3.

In some embodiments (not according to the claimed invention), a kit comprises reagents for blocking 5hmC. In some embodiments (not according to the claimed invention), a kit comprises a ShmC blocking group and a glucosyltransferase enzyme. In some embodiments (not according to the claimed invention), a ShmC blocking group is a uridine diphosphate (UDP)-sugar where the sugar is glucose or a glucose variant, and a glucosyltransferase enzyme is T4 bacteriophage β glucosyltransferase (βGT), T4 bacteriophage a-glucosyltransferase (αGT), and variants and analogs thereof.

In some embodiments (not according to the claimed invention), the kit comprises an oxidizing agent. In some embodiments (not according to the claimed invention), the oxidizing agent is a metal oxide. In some embodiments (not according to the claimed invention), the oxidizing agent is a metal (VI) oxo complex. In some embodiments (not according to the claimed invention), the metal oxide is selected from manganese oxide (MnO2), potassium ruthenate (K2RuO4) potassium perruthenate (KRuO4) and/or Cu(II)/TEMPO (copper(II) perchlorate and 2,2,6,6-tetramethylpiperidine-1-oxyl (TEMPO)).

In some embodiments (not according to the claimed invention), a kit comprises reagents for blocking 5fC in the nucleic acid sample. In some embodiments (not according to the claimed invention), a kit comprises an aldehyde reactive compound including, for example, hydroxylamine variants, hydrazine variants, and hyrazide variants as described herein. In some embodiments (not according to the claimed invention), a kit comprises a reducing agent such as sodium borohydride (NaBH4). In some embodiments (not according to the claimed invention), the kit comprises reagents for blocking 5caC.

In some embodiments (not according to the claimed invention), a kit comprises reagents for isolating a nucleic acid. In some embodiments (not according to the claimed invention), the nucleic acid is DNA or RNA. In some embodiments (not according to the claimed invention) the kit comprises reagents for isolating low-input DNA from a sample, for example cfDNA from blood, plasma, or serum.

In some embodiments (not according to the claimed invention), a kit includes one or more buffers or concentrated stock solutions thereof.

In some embodiments (not according to the claimed invention), a kit includes one or more reference materials (e.g., a nucleic acid preparation of known sequence and modification status.

### Compositions

In some embodiments (not according to the claimed invention), including as discussed elsewhere herein, the present disclosure provides a variety of compositions useful and/or used in performing reactions as described herein.

For example, in some embodiments (not according to the claimed invention), provided compositions may be or comprise a nucleic acid (e.g., an isolated and/or manipulated source nucleic acid), together with one or more components (e.g., reagents, buffers, etc) useful in performing one or more reaction(s) as described herein.

In some embodiments (not according to the claimed invention), provided compositions may include components useful for performing two or more reactions/steps as described herein; that is, in some embodiments (not according to the claimed invention), the present disclosure provides "one-pot" reactions (e.g., that do not require intervening separation steps) for two or more reactions as described herein. For example, in some embodiments (not according to the claimed invention), provided compositions may include reagents for one or more oxidation and/or reduction steps as described herein.

In some embodiments (not according to the claimed invention), provided compositions may comprise a nucleic acid and an oxidizing agent as described herein.

In some embodiments (not according to the claimed invention), provided compositions comprise a nucleic acid and a metal oxide. In some embodiments (not according to the claimed invention), compositions comprise a nucleic acid and a TET enzyme. In some embodiments (not according to the claimed invention), compositions comprise a nucleic acid and a reducing agent (e.g. pyridine borane and/or pic-borane (pic-BH3)). In some embodiments (not according to the claimed invention), compositions comprise a nucleic acid (e.g., a source nucleic acid and/or processed nucleic acid) and a reducing agent (e.g. pyridine borane and/or pic-borane (pic-BH3)). In some embodiments (not according to the claimed invention), compositions comprise a nucleic acid and one or more reagents suitable for blocking (e.g., a glucosyltransferase and glucose substrate). In some embodiments (not according to the claimed invention), compositions comprise a combination of one or more of a nucleic acid, an oxidizing agent, and a reducing agent.

In some embodiments (not according to the claimed invention), compositions as described herein (e.g., nucleic acid compositions) are at a pH below about 8; in some embodiments (not according to the claimed invention), such pH is below about 7; in some embodiments (not according to the claimed invention) such pH is below about 6. In some embodiments (not according to the claimed invention), compositions as described herein are within a pH range of about 5 to about 6. In some embodiments (not according to the claimed invention), compositions as described herein comprise an alcohol. In some embodiments (not according to the claimed invention), compositions as described herein comprise a salt. In some embodiments (not according to the claimed invention), compositions as described herein comprise a sodium salt. In some embodiments (not according to the claimed invention), compositions as described herein comprise an acid. In some embodiments (not according to the claimed invention), compositions as described herein comprise acetic acid and/or acetate.

In some embodiments (not according to the claimed invention), a provided composition is a combination or other admixture of components as described herein; in many embodiments (not according to the claimed invention) such composition is a liquid composition; in many embodiments (not according to the claimed invention) a provided composition (e.g., a liquid composition) comprises a buffer. In some embodiments (not according to the claimed invention), provided compositions are useful for detection (e.g., identification) of modified cytosines in nucleic acid (e.g., in a target nucleic acid, which in some embodiments (not according to the claimed invention) may be or be prepared from a source nucleic acid). In some embodiments (not according to the claimed invention), provided compositions may comprise reagents for identification of SmC and/or ShmC by methods described herein. Alternatively, or additionally, in some embodiments (not according to the claimed invention), compositions may contain reagents for detection (e.g., identification) of 5caC and/or SfC by methods described herein.

In some embodiments (not according to the claimed invention), a kit comprises a TET enzyme, a borane reducing agent and instructions for performing a method. In some embodiments (not according to the claimed invention), the TET enzyme is TET1 and the borane reducing agent is selected from one or more of the group consisting of pyridine borane, 2-picoline borane (picBH3), borane, sodium borohydride, sodium cyano borohydride, and sodium triacetoxyborohydride. In some embodiments, the TET1 enzyme is NgTetl or murine TETl and the borane reducing agent is pyridine borane and/or pic-BH3.

In some embodiments (not according to the claimed invention), a kit comprises reagents for blocking 5hmC. In some embodiments (not according to the claimed invention), a kit comprises a ShmC blocking group and a glucosyltransferase enzyme. In some embodiments (not according to the claimed invention), a ShmC blocking group is a uridine diphosphate (UDP)-sugar where the sugar is glucose or a glucose variant, and a glucosyltransferase enzyme is T4 bacteriophage β glucosyltransferase (βGT), T4 bacteriophage a-glucosyltransferase (αGT), and variants and analogs thereof.

In some embodiments (not according to the claimed invention), the kit comprises an oxidizing agent. In some embodiments (not according to the claimed invention), the oxidizing agent is a metal oxide. In some embodiments (not according to the claimed invention), the oxidizing agent is a metal (VI) oxo complex. In some embodiments (not according to the claimed invention), the metal oxide is selected from manganese oxide (MnO2), potassium ruthenate (K2RuO4) potassium perruthenate (KRuO4) and/or Cu(II)/TEMPO (copper(II) perchlorate and 2,2,6,6-tetramethylpiperidine-1-oxyl (TEMPO)).

In some embodiments (not according to the claimed invention), a kit comprises reagents for blocking 5fC in the nucleic acid sample. In some embodiments (not according to the claimed invention), a kit comprises an aldehyde reactive compound including, for example, hydroxylamine variants, hydrazine variants, and hyrazide variants as described herein. In some embodiments (not according to the claimed invention), a kit comprises a reducing agent such as sodium borohydride (NaBH4). In some embodiments (not according to the claimed invention), the kit comprises reagents for blocking 5caC.

In some embodiments (not according to the claimed invention), a kit comprises reagents for isolating a nucleic acid. In some embodiments (not according to the claimed invention), the nucleic acid is DNA or RNA. In some embodiments (not according to the claimed invention) the kit comprises reagents for isolating low-input DNA from a sample, for example cfDNA from blood, plasma, or serum.

### Exemplification

### Example 1: Materials and Methods

### Preparation of spike-in DNA

Detailed preparation protocols and sequences of CpG-methylated lambda DNA, 2kb-unmodified, and synthetic spike-in with SmC and ShmC modification can be found in previous publication (See, Liu et al., "Bisulfite-free direct detection of 5-methylcytosine and 5-hydroxymethylcytosine at base resolution", Nat. Biotechnol., 37, 2019). SfC spike-in was produced by an annealing and extension method with 5-formylcytidine-5'-triphosphate (5-fCTP, TriLink BioTechnologies). ScaC spike-in was produced by PCR amplification from the pNIC28-Bsa4 plasmid (Addgene, cat. no. 26103), then methylated with M.SssI enzyme (NEB) and oxidized with two round of mTetlCD treatment. Sequences of 5fC and ScaC spike-ins were listed in table S3.

### mESCs culture and genomic DNA extraction

E14 mouse embryonic stem cells (mESCs) were gifted from Professor Skirmantas Kriaucionis and cultured on gelatin-coated plates in Dulbecco's Modified Eagle Medium (DMEM) (Invitrogen) supplemented with 15% FBS (GIBCO), 2 mM L-glutamine (Gibco), 1% non-essential amino acids (Gibco), 1% penicillin/streptavidin (Gibco), 0.1 mM β-mercaptoethanol (Sigma), 1000 units/mL LIF (Millipore), 1 µM PD0325901 (Stemgent), and 3 µM CHIR99021 (Stemgent). mESCs were maintained at 37°C and 5% CO2 and passaged every 2 days. The genomic DNA was prepared by cell harvesting with centrifugation for 5 min at 1000x g and room temperature, and DNA extraction with Quick-DNA Plus kit (Zymo Research) according to the manufacturer's protocol.

### Preparation of mESC gDNA and sequencing library construction

mESC gDNA was spiked with 0.5% of methylated lambda DNA, 0.025% of 2kb-unmodified and 0.025% of 2kb-caC spike-in controls. For CAPS approach, gDNA was fragmented by Covaris M220 instrument and size-selected to 200-400 bp using Ampure XP beads (Beckman Coulter). For other approaches, gDNA was fragmented and size-selected to 300-500 bp. 0.01% of synthetic oligo withN5mCNN /N5hmCNN sequences and 0.01% of synthetic oligo with SfC modifications were added after size-selection. 100 ng of fragmented DNA was used for end-repair/A-tailing and ligation of NEBNext Adaptor (NEB) with KAPA Hyper kit (KAPA) according to the manufacturer's protocol. The uracil in the loop of NEBNext Adaptor was removed by adding 3 µL of USER enzyme (NEB) to the ligation reaction and incubating for 15 min at 37°C. Then the reaction was purified with 0.8X Ampure XP beads according to the manufacturer's protocol. For CAPS approach, 80% of acetonitrile: H2O was used instead of 80% ethanol: H2O during beads purification step.

### TET Assisted Pyridine borane Sequencing with β-glucosyltransferase blocking (TAPSβ)

mTetlCD was expressed and purified as previously described (See, Liu et al., "Bisulfite-free direct detection of 5-methylcytosine and 5-hydroxymethylcytosine at base resolution", Nat. Biotechnol., 37, 2019). Ligated DNA was added to a 50 µL reaction containing 50 mM HEPES buffer (pH 8), 25 mM MgCl2, 200 µM UDP-Glc (NEB) and 10 U of β-glucosyltransferase (Thermo Fisher) for 1 h at 37°C. ShmC blocked DNA was purified with Ampure XP and then incubated in 50 µL oxidation reaction containing 50 mM HEPES buffer (pH 8.0), 100 µM ammonium iron (II) sulfate, 1 mM a-ketoglutarate, 2 mM ascorbic acid, 1 mM dithiothreitol, 100 mM NaCl, 1.2 mM ATP and 4 µM mTetlCD for 80 min at 37°C. Then 0.8 U of Proteinase K (NEB) was added to the reaction and incubated for 1 h at 50°C. Oxidized DNA was purified with Ampure XP beads and then input into another round of TET oxidation in order to achieve complete oxidation. The double-oxidized DNA was added to a 50 µL reaction containing 600 mM NaAc (pH = 4.3) and 1 M pyridine borane (Alfa Aesar). The reaction was incubated at 37°C and 850 rpm in a ThermoMixer (Eppendorf) for 16 hours and purified by Zymo-IC column (Zymo Research) with Oligo Binding Buffer (Zymo Research).

### Chemical Assisted Pyridine borane Sequencing (CAPS)

Potassium ruthenate (K2RuO4) was prepared as previously described by Zeng et. al (See, for example, Zeng et al. "Bisulfite-Free, Nanoscale Analysis of 5-Hydroxymethylcytosine at Single Base Resolution" J. Am. Chem. Soc., 140, 2018) and stored in -20°C refrigerator as 10x oxidant. 2 M 2-methylpyridine borane (Sigma) was prepared by dissolving the solid in EtOH. Before ShmC oxidation, ligated DNA was purified with Micro Bio-Spin P-6 SSC column (Bio-Rad, washed 5 times with water before use). The purified DNA was denatured in 20 µL solution containing 0.05 M NaOH for 30 min at 37°C. 10x oxidant was diluted to 1x with distilled water and 2.5 µL of 1x oxidant was added to the denatured DNA. The oxidation reaction was incubated at 37°C and 850 rpm in a ThermoMixer for 1 hour. Then additional 2.5 µL of 1x oxidant was added to the same reaction and incubated at 37°C and 850 rpm in a ThermoMixer for another hour. The oxidized DNA was purified by a Bio-Rad Micro Bio-Spin P-6 SSC column, and added to a reaction containing 0.3 M MES (Sigma, pH 5.2) and 0.2 M 2-methylpyridine borane. The reaction was incubated at 37°C and 850 rpm in a ThermoMixer for 2 hours and purified by Zymo-IC column with Oligo Binding Buffer.

### Quantification of 5mC, 5hmC and 5fC level by HPLC-MS/MS

Control and oxidized genomic DNA samples were digested into nucleosides and then analyzed with HPLC-MS/MS as previously described (See, Liu et al., "Bisulfite-free direct detection of 5-methylcytosine and 5-hydroxymethylcytosine at base resolution", Nat. Biotechnol., 37, 2019).

### Pyridine borane Sequencing (PS)

Ligated DNA was added to a 50 µL reaction containing 0.6 M NaAc (pH = 4.3) and 1 M pyridine borane. The reaction was incubated at 37°C and 850 rpm in a ThermoMixer for 16 hours and purified by Zymo-IC column with Oligo Binding Buffer.

### Pyridine borane Sequencing for Carboxylcytosine (PS-c)

Ligated DNA was added to a 50 µL reaction containing 10 mM O-ethylhydroxylamine (Aldrich) and 100 mM MES buffer (pH 5.0). The reaction was incubated at 37°C and 850 rpm for 4 hours in a Thermomixer and purified with Ampure XP beads. SfC-blocked DNA was then added to a 50 µL reaction containing 0.6 M NaAc (pH = 4.3) and 1 M pyridine borane. The reaction was incubated at 37°C and 850 rpm in a ThermoMixer for 16 hours and purified by Zymo-IC column with Oligo Binding Buffer.

### PCR amplification of converted DNA and sequencing

Converted DNA was amplified with KAPA HiFi HotStart Uracil+ ReadyMix PCR Kit (KAPA) for 4 cycles according to the manufacturer's protocol with minor modification. Dual index primers in NEBNext Multiplex Oligos for Illumina was used instead of the Library Amplification Primer Mix. PCR product was purified with 1X Ampure XP beads and quantified with Qubit dsDNA HS Assay Kit (ThermoFisher). When starting with 100 ng of fragmented DNA for library construction, typical final library yield should be > 30 nM after 4 cycles of PCR amplification. Libraries were sequenced on NovaSeq 6000 (150 bp paired-end) with no PhiX added.

### Blocking of 5fC with Reducing Agent

37.8 mg of NaBH4 was dissolved in 1 mL water to prepare fresh 1 M solution. 5 µL of the solution was added to 15 µL DNA and incubated at room temperature for 1 hour in dark and the lid was opened every 15 min. Then 10 µL of 750 mM NaAc (pH=5) was added to quench the reaction at room temperature for 10 min then purified with Zymo IC column and Oligo binding buffer. The purified DNA was incubated with 0.6 M MES (pH=5.2) and 0.5 M Pic-borane at room temperature for 2 hours. The reaction was purified with Zymo IC column and Oligo binding buffer.

### Data preprocessing

Sequencing reads were trimmed with Trim Galore! v0.3.1 (https://www.bioinformatics.babraham.ac.uk/projects/trim_galore/) to remove adapters and low-quality bases. Trimmed reads were mapped to a genome combining spike-in sequences and the mm9 mouse genome using BWA mem v.0.7.12 (See, for example, Li et al., "Fast and accurate short read alignment with Burrows-Wheeler transform", Bioinformatics, 25, 2009). PCR duplicates were removed using MarkDuplicate function of Picard v2.3.0 (http://broadinstitute.github.io/picard/). Reads with MAPQ < 10 were excluded from methylated site calling. Modified bases were called by asTair v3.3.1 (See, Liu et al., "Bisulfite-free direct detection of 5-methylcytosine and 5-hydroxymethylcytosine at base resolution", Nat. Biotechnol., 37, 2019). Raw signals were calculated as the ratio between C and C+T at each site. Regions known to be prone to mapping artifacts (https://sites.google.com/site/anshulkundaje/projects/blacklists) (See, Consortium et al., "An integrated encyclopedia of DNA elements in the human genome", Nature, 489, 2012; and Amemiya et al., "The ENCODE Blacklist: Identification of Problematic Regions of the Genome", Sci. Rep.., 9, 2019) and known single nucleotide variants (http://epigenetics.hugef-research.org/data.php) (See, Incarnato et al., "High-Throughput single nucleotide variant discovery in E14 mouse embryonic stem cells provides a new reference genome assembly", Genomics, 104, 2014) of the E14 cell line were used to exclude those overlapping sites from subsequent analysis. The mapping rate was calculated as the ratio between the number of properly mapped read pairs (MAPQ > 10) and the number of trimmed read pairs by Samtools (See, Handsaker et al., "Genome Project Data Processing, The Sequence Alignment/Map format and SAMtools", Bioinformatics, 25, 2009). The base quality was visualized by the phred function of asTair (See, Liu et al., "Bisulfite-free direct detection of 5-methylcytosine and 5-hydroxymethylcytosine at base resolution", Nat. Biotechnol., 37, 2019).

### Published datasets

We used the following published datasets: TAPS data and WGBS data (GSE112520) (See, Liu et al., "Bisulfite-free direct detection of 5-methylcytosine and 5-hydroxymethylcytosine at base resolution", Nat. Biotechnol., 37, 2019), oxBS-seq data (GSE112875) (See, for example, Liu et al. "5-Methylcytosine-Specific Amplification and Sequencing" J. Am. Chem. Soc. 142, 2020), TAB-seq data (GSE36173) (See, for example, Yu et al. "Base-resolution analysis of 5-hydroxymethylcytosine in the mammalian genome" Cell, 149, 2012) and ACE-seq data (GSE116016.) (See, for example, Schutsky et al. "Nondestructive, base-resolution sequencing of 5-hydroxymethylcytosine using a DNA deaminase" Nat. Biotechnol., 36, 2018). The TAB-seq data were reprocessed to obtain the full list of modified and unmodified sites. The sequencing reads were downloaded and trimmed by Trim Galore! v0.3.1 (https://www.bioinformatics.babraham.ac.uk/projects/trim_galore/). The trimmed reads were aligned to mm9 using bismark v0.18.1 (See, Krueger et al., "Bismark: a flexible aligner and methylation caller for Bisulfite-Seq applications", Nat. Methods, 27, 2012) and bowtie v2.2.1 (See, Langmead et al., "Fast gapped-read alignment with Bowtie 2", Nat. Methods, 9, 2012). PCR duplicates were removed from the mapped bam file using MarkDuplicate function of Picard v2.3.0 (http://broadinstitute.github.io/picard/). The reads with over three non-conversion sites were filtered using the filter_non_conversion function of bismark as previously described (See, for example, Yu et al. "Base-resolution analysis of 5-hydroxymethylcytosine in the mammalian genome" Cell, 149, 2012). The methylation sites were called by bismark_methylation_extractor and masked by intersectBed (bedtools v2.25.0) (See, Quinlan et al., "BEDTools: a flexible suite of utilities for comparing genomic features", Bioinformatics, 26, 2010) to remove sites in regions known to be prone to mapping artifacts.

### Pairwise comparisons of TAPSβ

The three replicates of oxBS-seq results were pooled together for the correlation analysis. Sites with a minimal coverage of ten reads were used for the correlation analysis between TAPSβ and oxBS-seq. The Pearson correlation coefficient (Pearson's r) was calculated by using R function cor. The scatterplot with smoothed densities color representation (Fig. 1D) was visualized using function smoothScatter in R.

### Coverage analysis of CAPS and ACE-seq

The CpG island annotation was downloaded from UCSC (See, Rosenbloom et al., "ENCODE data in the UCSC Genome Browser: year 5 update", Nucleic Acids Res., 41, 2013). Each CpG island was evenly binned into ten windows. The 4-kb flanking regions were binned into twenty windows. The coverage was defined as the sum of modified and unmodified reads at each site. The average coverage was calculated by Bedtools map. Given that the overall coverage of CAPS was higher than ACE-seq, the coverage at each site was normalized by the ratio of overall coverage between the two datasets.

### Estimation of 5hmC using maximum likelihood

To estimate ShmC levels from TAPS and TAPSβ, the maximum likelihood methylation levels (MLML) estimation method was applied on sites with a minimum coverage of 5. The sites with at least one conflict were excluded from subsequent analysis. The average levels of unmodified C, SmC and ShmC estimated by MLML were tiled by 1-kb bins and visualized by R package Ternary (https://github.com/ms609/Ternary/tree/1.1.4) (Fig. 3A).

### Pairwise comparisons of CAPS

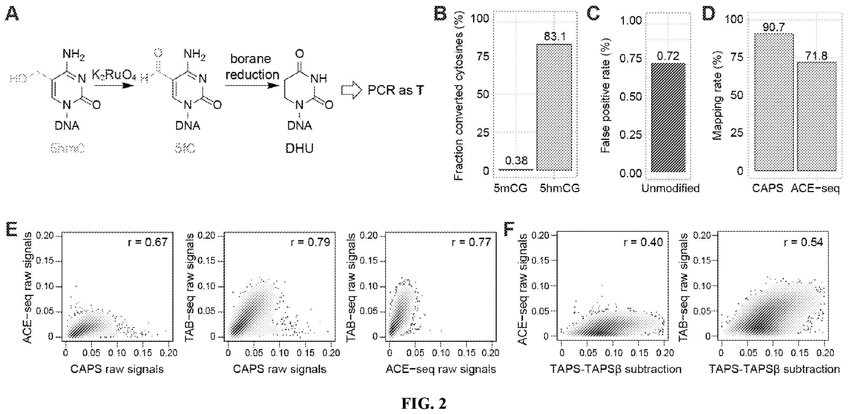

To compare CAPS withACE-seq and TAB-seq, the raw ShmCG signals, i.e. C/(C+T), were calculated within 10-kb genomic bins (Fig. 2F) as previously defined (See, for example, Schutsky et al. "Nondestructive, base-resolution sequencing of 5-hydroxymethylcytosine using a DNA deaminase" Nat. Biotechnol., 36, 2018). The 10-kb raw signal of TAPS-TAPSβ subtraction was calculated as the average estimated ShmC levels from the MLML output.

### Genomic view

To view the methylation levels on genomes, the methylation calling output was transferred to the bigwig format by bedGraphToBigWig (See, Kent et al., "BigWig and BigBed enabling browsing of large distributed datasets", Bioinformatics, 26, 2010) and visualized by the Integrative Genomics Viewer (See, Robinson et al., "Integrative genomics viewer", Nat. Biotechnol., 29, 2011) on the mm9 genome.

### Statistical test of 5hmC and 5fC

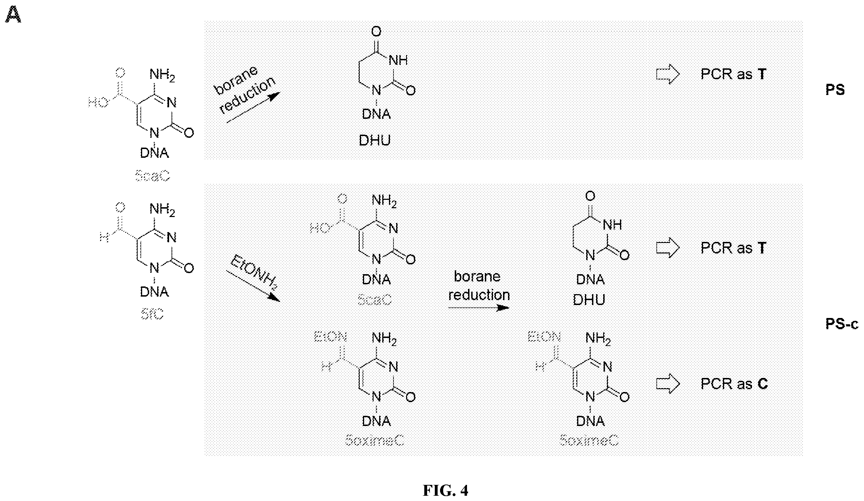

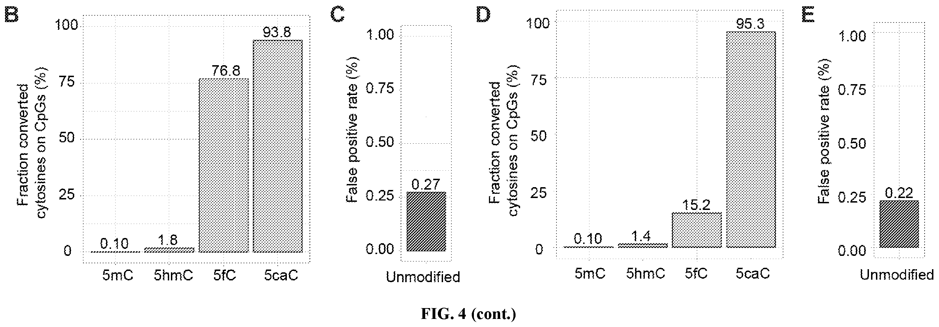

We used the binomial test (See, for example, Yu et al. "Base-resolution analysis of 5-hydroxymethylcytosine in the mammalian genome" Cell, 149, 2012) to call ShmC at sites with the minimal coverage of five reads. The probability P of the binomial distribution was the false positive rate (0.0072) of CAPS, calculated from the unmodified control DNA (Fig. 2C). Cytosines with Benjamini-Hochberg (BH) adjusted p-value < 0.05 were used for downstream analysis. To estimate the number of 5fC modified CpG sites, this binomial test was applied to PS by using the false positive rate of 0.0027 (Fig. 4C).

### Quantifying enrichment of called 5hmCGs in genomic regulatory elements

The list of putative genomic regulatory elements was downloaded (https://github.com/gireeshkbogu/chromatin_states_chromHMM_mm9) (See, for example, Bogu et al., "Chromatin and RNA Maps Reveal Regulatory Long NONcoding RNAs in Mouse", Mol. Cell. Biol., 36, 2015). This list was predicted based on the ENCODE data (See, for example, Shen et al., "A map of the cis-regulatory sequences in the mouse genome", Nature, 488, 2012) by ChromHMM (See, for example, Ernst et al. "ChromHMM: automating chromatin-state discovery and characterization" Nat. Methods, 9, 2012). The high-confidence ShmCG sites (BH-adjusted p-value < 0.05 and coverage ≥ 5 reads) were annotated using Bedtools intersect. The number of ShmCG sites fell into each category was counted (Fig. 3C). To investigate the enrichment of ShmCG in each element class, a set of CG sites was sampled for ten times to generate a background distribution of CG sites across element categories. The number of ShmCGs or random CGs was normalized by the genomic coverage of corresponding regulatory elements.

### Code availability

The asTair package is available at https://pypi.org/project/asTair/. The in-house analysis scripts are available at https://github.com/zhiyhu/CAPS-paper.

### Example 2: TAPSβ analysis of mouse genomic DNA

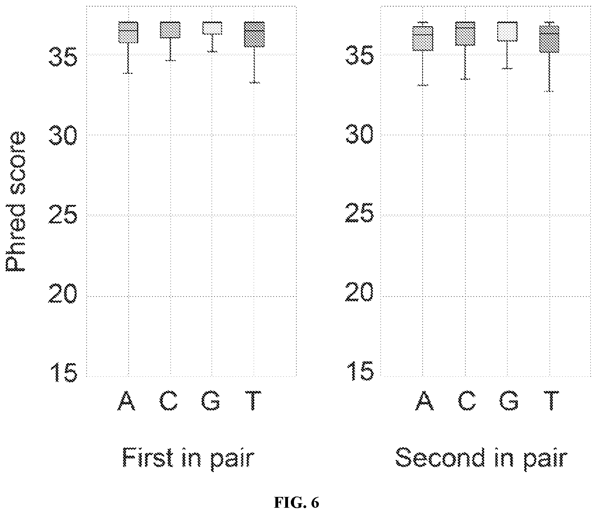

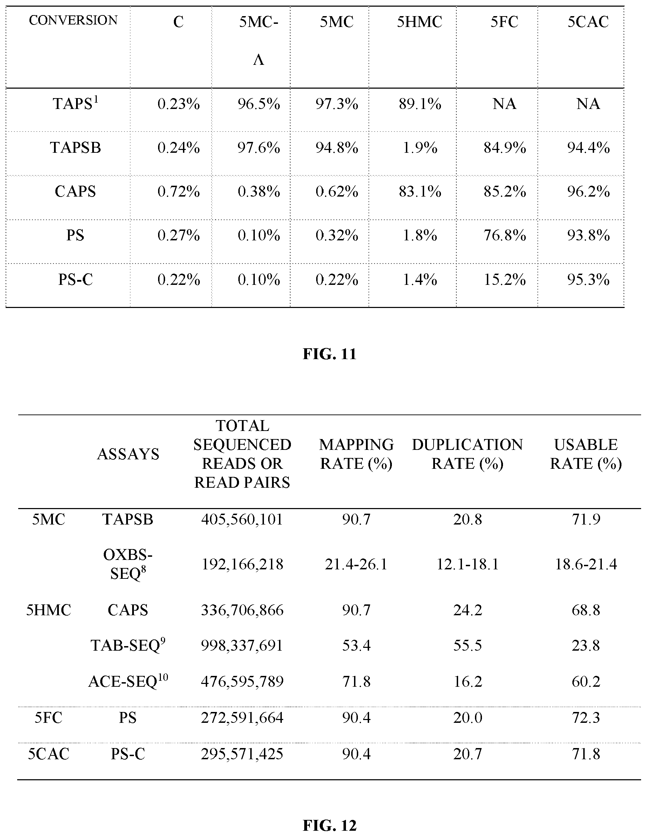

The present example confirms that TAPSβ can be applied to mESC genomic DNA (gDNA). mESC gDNA was treated with β-glucosyltransferase (βGT)to glucosylate and block 5hmC. This was followed by TET oxidation and borane reduction on SmC (Fig. 1A) (See, Liu et al., "Bisulfite-free direct detection of 5-methylcytosine and 5-hydroxymethylcytosine at base resolution", Nat. Biotechnol., 37, 2019). Samples were then validated with spike-in controls with known modifications by high-throughput sequencing. High SmC conversion rate (97.6% in CpG-methylated lambda DNA, Fig. 1B) and low false positive rate (0.24% conversion rate on unmodified C, Fig. 1C) were achieved in TAPSβ, which are close to previous TAPS results in model DNA (96.5% and 0.23%, respectively). 5hmC showed only 1.9% conversion rate in TAPSβ (Fig. 1B) compared to 89.1% in TAPS (See, Liu et al., "Bisulfite-free direct detection of 5-methylcytosine and 5-hydroxymethylcytosine at base resolution", Nat. Biotechnol., 37, 2019). The other two minor cytosine modifications SfC and ScaC also showed high conversion rate (84.9% and 94.4% respectively, Fig. 11).. TAPSβ showed excellent sequencing quality scores at cytosine/guanine (Fig. 6). Good correlation between TAPSβ and published 5mC data of mESCs by oxBS-seq was observed (Pearson's r = 0.72, Fig. 1D), although TAPSβ showed a much higher mapping rate (90.7%, table S2) than oxBS-seq (21.4-26.1%, table S2) (See, for example, Liu et al. "5-Methylcytosine-Specific Amplification and Sequencing" J. Am. Chem. Soc. 142, 2020). These results confirm that TAPSβ works well in both gDNA and model DNA (See, Liu et al., "Bisulfite-free direct detection of 5-methylcytosine and 5-hydroxymethylcytosine at base resolution", Nat. Biotechnol., 37, 2019).

### Example 3: CAPS analysis of mouse genomic DNA

The present example demonstrates that CAPS can be applied to mESC genomic DNA (gDNA) with a ruthenate oxidizing agent. Nucleic acids can be treated with chemical oxidizing reagents to oxidize 5hmC to SfC, which can then be converted to DHU by borane reduction (Fig. 2A). The present example makes use of a potassium ruthenate (K2RuO4), which was used in chemical-assisted C-to-T conversion of 5hmC sequencing (hmC-CATCH) and reported to be more oxidative and less DNA damaging (See, for example, Zeng et al. "Bisulfite-Free, Nanoscale Analysis of 5-Hydroxymethylcytosine at Single Base Resolution" J. Am. Chem. Soc., 140, 2018) than potassium perruthenate (KRuO4), which was previously used in oxBS-seq and TAPS . The K2RuO4 oxidation protocol for CAPS was employed a commonly used double-stranded DNA library preparation method , rather thana complicated single-strand protocol. Additionally, a uracil-containing loop-structured NEBNext Adaptor was used in the DNA ligation step of the library preparation. Subsequent treatment with USER enzyme (a mix of UDG and Endo VIII) opened the loop, leaving 3' and 5' phosphate ends that could protect the ligated DNA from oxidative damage (See, for example, Wang et al. "Bisulfite-Free, single base-resolution analysis of 5-hydroxymethylcytosine in genomic DNA by chemical-mediated mismatch" Chem Sci, 10, 2019).Double oxidation was performed on the ligated DNA by adding additional oxidant to the original oxidation reaction, improving the conversion rate of 5hmC to 5fC from 82.8% to 97.2% as measured by HPLC-MS/MS (fig. S2). Validation of CAPS with spike-in controls by high-throughput sequencing showed 83.1% ShmC-to-T conversion rate (Fig. 2B) and 0.72% false positive rate (Fig. 2C). These numbers are comparable to the ShmC-to-T conversion rate and false positive rate reported in hmC-CATCH (~80% (without pull-down) and 0.6-1%, respectively) (See, for example, Zeng et al. "Bisulfite-Free, Nanoscale Analysis of 5-Hydroxymethylcytosine at Single Base Resolution" J. Am. Chem. Soc., 140, 2018).

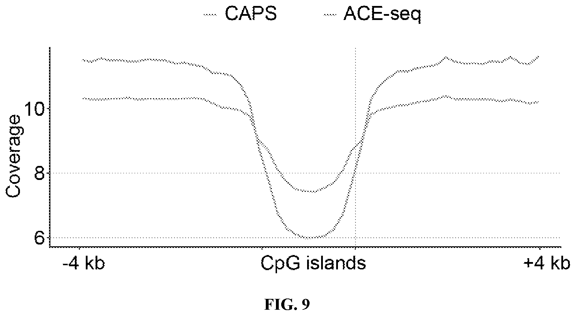

CAPS was then applied mESC gDNA to detect 1,762,287 ShmC-modified sites. In comparison to two commonly used sequencing methods in the field, TAB-Seq and ACE-Seq, CAPS displayed higher mapping rate, base quality, and coverage (Fig. 2D and Fig. 9, Fig. 12), while showing good correlation with previously published datasets (Pearson's r = 0.79 with TAB-seq and 0.67 with ACE-seq, Fig. 2E). On the other hand, 5hmC obtained from TAPS-TAPSβ subtraction showed an abnormal distribution of modification levels with significantly lower correlation (Pearson's r = 0.54 with TAB-seq and 0.40 with ACE-seq, Fig. 2F), demonstrating that the subtraction-free method is superior for 5hmC profiling, especially given 5hmC exists in much lower abundance than SmC in most non-neuronal tissues and cell lines (See, Ito et al., "Tet proteins can convert 5-methylcytosine to 5-formylcytosine and 5-carboxylcytosine", Science, 333, 2011), including in mESCs (fig. S2A).

### Example 4: Comparison of detection methods

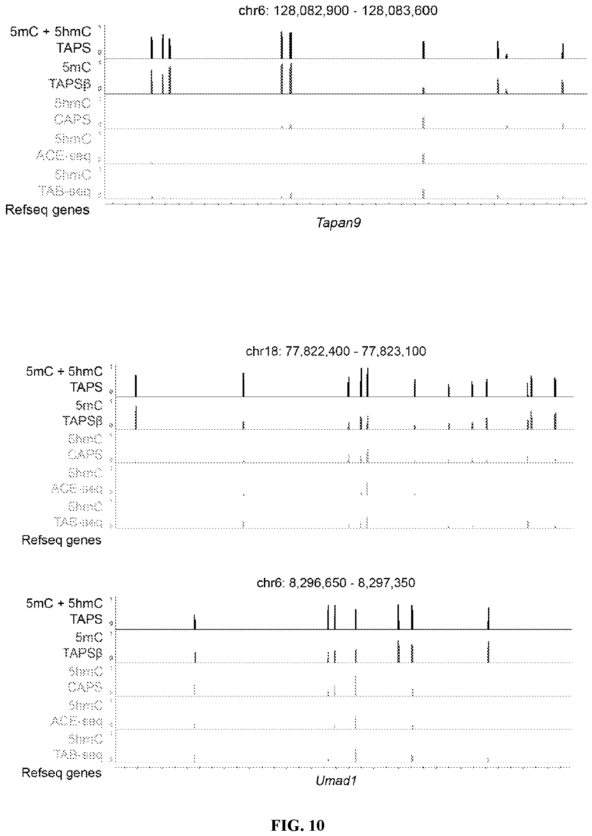

The present example demonstrates that subtraction free methods, such as CAPS and TAPSβ, offer improved accuracy over subtraction-based methods. Ternary plots of C, SmC and 5hmC distribution in mESCs were generated for TAPSβ and CAPS, TAPS and TAPSβ (subtraction), WGBS and ACE-Seq, and WGBS and TAB-Seq (Fig. 3A). Combination of TAPSβ and CAPS showed a similar pattern to WGBS with TAB-seq or ACE-seq while, TAPS-TAPSβ subtraction overestimated 5hmC sites. Certain data were plotted to show results from different approaches, demonstrating that CAPS detected 5hmC sites consistent with TAB-seq and ACE-seq (Fig. 3B and fig. 10). Distribution of 5hmC varied across genomic regulatory elements (Fig. 3C) (See, for example, Ernst et al. "ChromHMM: automating chromatin-state discovery and characterization" Nat. Methods, 9, 2012; Shen et al., "A map of the cis-regulatory sequences in the mouse genome", Nature, 488, 2012; and Bogu et al., "Chromatin and RNAMaps Reveal Regulatory Long NONcoding RNAs in Mouse", Mol. Cell. Biol., 36, 2015), with particular enrichment at enhancers and insulators (See, for example, Kim et al., "CTCF as a multifunctional protein in genome regulation and gene expression", Exp. Mol. Med., 47, 2015), where CTCF-binding sites were enriched (Fig. 3D). This result is consistent with previous findings that ShmCs are enriched in enhancers and CTCF-binding sites (See, for example, Zeng et al. "Bisulfite-Free, Nanoscale Analysis of 5-Hydroxymethylcytosine at Single Base Resolution" J. Am. Chem. Soc., 140, 2018; and Yu et al., "Base-resolution analysis of 5-hydroxymethylcytosine in the mammalian genome", Cell, 149, 2012).

### Example 5: Exemplary Detection of 5mC (not according to the claimed invention)

In some embodiments, the present disclosure provides a method for identifying SmC or 5hmC in a source nucleic acid comprising steps of
a. providing a source nucleic acid;
b. modifying the source nucleic acid comprising the steps of:
   i. adding a blocking group to the 5hmC in the source nucleic acid;
   ii. converting the SmC in the source nucleic acid to 5caC and/or 5fC; and
   iii. converting the 5caC and/or 5fC to DHU to provide a processed nucleic acid; and
c. detecting the sequence of the processed nucleic acid;
wherein the presence of a cytosine (C) to thymine (T) transition in the sequence of the processed nucleic acid compared to a reference nucleic acid indicates the presence of SmC in a source nucleic acid.

In some embodiments of a method for identifying SmC in a source nucleic acid, the method provides a quantitative measure for the frequency the of SmC modification at each location where the modification was identified in the source nucleic acid. In some embodiments, the percentages of the T at each transition location provide a quantitative level of 5mC at each location in a source nucleic acid.

In order to identify 5mC in a source nucleic acid without including ShmC, 5hmC in a source nucleic acid is blocked so that it is not subject to conversion to 5caC and/or 5fC. In some embodiments, 5hmC bases in a source nucleic acid are rendered non-reactive to subsequent steps by adding a blocking group to 5hmC. In some embodiments, a blocking group is a sugar, including a modified sugar, for example glucose or 6-azide-glucose (6-azido-6-deoxy-D-glucose). In some embodiments, a sugar blocking group is added to hydroxymethyl group of 5hmC by contacting a source nucleic acid with uridine diphosphate (UDP)-sugar in the presence of one or more glucosyltransferase enzymes.

In some embodiments, a glucosyltransferase is a T4 bacteriophage β-glucosyltransferase (βGT) a T4 bacteriophage α-glucosyltransferase (αGT), or variants and analogs thereof. In some embodiments, βGT catalyzes a chemical reaction in which a beta-D-glucosyl (glucose) residue is transferred from UDP-glucose to a 5-hydroxymethylcytosine residue in a source nucleic acid.

In some embodiments, a blocking group comprising a glucose moiety (e.g., a beta-D-glucosyl residue) is added to 5hmC to yield glucosyl 5-hydroxymethyl cytosine. In some embodiments, a blocking group can be a sugar (e.g., a natural or modified sugar) that is a substrate of the glucosyltransferase enzyme and blocks the subsequent conversion of the 5hmC to 5caC and/or 5fC. In some embodiments, a step of converting SmC in a source nucleic acid to 5caC and/or 5fC is accomplished by the methods provided herein (e.g., oxidation with a TET enzyme). In some embodiments, a step of converting 5caC and/or 5fC to DHU is accomplished by methods provided herein (e.g., borane reduction).

### Example 6: Exemplary Detection of SmC or 5hmC (together) (not according to the claimed invention)

In some embodiments, the present disclosure provides a method for identifying SmC or 5hmC in a source nucleic acid comprising steps of
a. providing a source nucleic acid;
b. modifying the nucleic acid comprising the steps of:
   i. converting SmC and 5hmC in the source nucleic acid to 5-carboxylcytosine (5caC) and/or 5fC; and
   ii. converting the 5caC and/or 5fC to DHU to provide a processed nucleic acid; and
c. detecting the sequence of the processed nucleic acid;
wherein the presence of a cytosine (C) to thymine (T) transition in the sequence of the processed nucleic acid compared to a reference nucleic acid indicates the presence of SmC or 5hmC in a source nucleic acid.

In embodiments, the method provides a quantitative measure for the frequency the of SmC or 5hmC modifications at each location where the modifications were identified in the target nucleic acid. In some embodiments, the method detects SmC and 5hmC at particular locations, but does not distinguish between each cytosine modification. Rather, both SmC and 5hmC are converted to DHU.

### Example 7: Exemplary Detection of SmC and 5hmC (not according to the claimed invention)

In some embodiments, the present disclosure provides a method for identifying SmC and identifying 5hmC in a source nucleic acid by (i) performing a method for identifying SmC on a first nucleic acid described herein (e.g., a source nucleic acid), and (ii) performing a method for identifying 5mC or 5hmC on a second nucleic acid described herein (e.g., a source nucleic acid). In some embodiments, a location of SmC is provided by (i). In some embodiments, a location of 5hmC is provided by comparing the results of (i) and (ii), wherein a C to T transition detected in (ii) but not in (i) provides the location of 5hmC in a nucleic acid. In some embodiments, the first and second nucleic acids are derived from the same nucleic acid (e.g., a source nucleic acid). For example, in some embodiments the first and second nucleic acids may be separate aliquots taken from a sample comprising a nucleic acid (e.g., a source nucleic acid).

In some embodiments, SmC and 5hmC are converted to 5fC and 5caC before conversion to DHU, such that existing 5fC and 5caC in the DNA sample will be detected as SmC and/or 5hmC. In some embodiments, due to low levels of 5fC and 5caC in a source nucleic acid under normal conditions, native 5fC and 5caC measurements will often be negligible. In some embodiments, a blocking step on 5fC and/or 5caC (e.g., hydroxylamine conjugation and/or EDC coupling) can be employed prior to conversion of SmC and 5hmC to DHU.

In some embodiments, a method described herein identifies locations of 5hmC in a nucleic acid (e.g., a source nucleic acid) through comparison of 5mC locations with locations of 5mC or 5hmC (together). In some embodiments, a method described herein identifies locations of 5hmC in a nucleic acid (e.g. a source nucleic acid) directly (e.g., through a subtraction-free method). Thus, in some embodiments the disclosure provides a method for identifying 5hmC in a nucleic acid comprising steps of:
a. providing a source nucleic acid;
b. modifying the source nucleic acid comprising the steps of:
   i. converting 5hmC in the source nucleic acid to 5caC and/or 5fC; and
   ii. converting the 5caC and/or 5fC to DHU to provide a processed nucleic acid;
c. detecting the sequence of the processed nucleic acid;
wherein a C to T transition in the sequence of the processed target nucleic acid compared to the source nucleic acid provides the location of a 5hmC in the source nucleic acid.

In some embodiments, the step of converting the 5hmC to 5fC comprises oxidizing 5hmC to 5fC by contacting a nucleic acid with a metal oxide. In some embodiments, a metal oxide is potassium perruthenate (KRuO4) (as described in Science. 2012, 33, 934-937 and WO2013017853); Cu(II)/TEMPO (copper(II) perchlorate and 2,2,6,6-tetramethylpiperidine-1-oxyl (TEMPO)) (as described in Chem. Commun., 2017,53, 5756-5759 and WO2017039002); or potassium ruthenate (K2RuO4) (See, for example, Zeng et al. "Bisulfite-Free, Nanoscale Analysis of 5-Hydroxymethylcytosine at Single Base Resolution" J. Am. Chem. Soc., 140, 2018). In some embodiments, 5fC in a nucleic acid (e.g., a source nucleic acid and/or processed nucleic acid) is then converted to DHU by one or more methods disclosed herein, e.g., by a borane reduction.

### Example 8: Exemplary Detection of 5fC or 5caC (not according to the claimed invention)

In some embodiments, the present disclosure provides a method for identifying 5caC or 5fC in source nucleic acid comprising steps of:
a. providing a source nucleic acid;
b. converting 5caC and/or 5fC to DHU to provide a processed nucleic acid;
c. optionally amplifying the copy number of the processed target nucleic acid;
d. detecting the sequence of the processed nucleic acid;
wherein a C to T transition in the sequence of the processed nucleic acid compared to the source nucleic acid provides a location of a 5caC and/or 5fC in the source nucleic acid.

In some embodiments, a method for identifying 5fC and/or 5caC provides the location of 5fC and/or ScaC, but does not distinguish between the two cytosine modifications. Rather, in some embodiments, both 5fC and 5caC are converted to DHU, which is detected by methods described herein. In some embodiments, assessments as described herein may include methods for identifying both 5fC and 5caC through pic/pyridine borane reduction and sequencing (e.g., PS method).

### Example 9: Exemplary Detection of 5caC (not according to the claimed invention)

In some embodiments, the disclosure provides a method for identifying 5caC in a source nucleic acid comprising steps of:
a. providing a nucleic acid sample comprising a source nucleic acid;
b. blocking 5fC in the source nucleic acid, e.g. by adding a blocking group;
c. converting 5caC to DHU to provide a processed nucleic acid;

a. optionally amplifying the copy number of the processed nucleic acid; and
b. detecting the sequence of the processed nucleic acid;
wherein a C to T transition in the sequence of the processed nucleic acid compared to the source nucleic acid provides a location of 5caC in the source nucleic acid.

In some embodiments, a method for identifying 5caC in a source nucleic acid provides a quantitative measure for the frequency of 5caC modifications at each location where the modification was identified in the source nucleic acid. In some embodiments, percentages of T at each transition location provide a quantitative level of 5caC at each location in the source nucleic acid. In some embodiments, assessments as described herein may include methods for identifying 5caC through blocking of 5fC, followed by pic/pyridine borane reduction and sequencing (e.g., PS-C method).

In some embodiments of this method, 5fC is blocked (and SmC and 5hmC are not converted to DHU), allowing identification of 5caC in the source nucleic acid. In some embodiments, adding a blocking group to the 5fC in the source nucleic acid comprises contacting the nucleic acid with an aldehyde reactive compound including, for example, hydroxylamine variants, hydrazine variants, and hydrazide variants. Hydroxylamine variantsinclude ashydroxylamine; hydroxylamine hydrochloride; hydroxylammonium acid sulfate; hydroxylamine phosphate; O-methylhydroxylamine; O-hexylhydroxylamine; O-pentylhydroxylamine; O-benzylhydroxylamine; and particularly, O-ethylhydroxylamine (EtONH2), O-alkylated or O-arylated hydroxylamine, acid or salts thereof. Hydrazine variantsinclude N-alkylhydrazine, N-arylhydrazine, N- benzylhydrazine, N,N-dialkylhydrazine, N,N-diarylhydrazine, N,N-dibenzylhydrazine, N,N-alkylbenzylhydrazine, N,N-arylbenzylhydrazine, and N,N-alkylarylhydrazine. Hydrazide variantsinclude -toluenesulfonylhydrazide, N-acylhydrazide, N,N-alkylacylhydrazide, N,N-benzylacylhydrazide, N,N-arylacylhydrazide, N-sulfonylhydrazide, N,N-alkylsulfonylhydrazide, N,N-benzylsulfonylhydrazide, and N,N-arylsulfonylhydrazide. In some embodiments, blocking of 5fC can comprise protection or derivatization through a chemical reaction. In some embodiments, blocking of 5fC comprises contacting a nucleic acid with a reducing agent and converting 5fC to 5hmC. In some embodiments, subsequent borane reduction, amplification, and/or sequencing of the nucleic acid (e.g. a processed nucleic acid) would produce a C to T transition only for ScaC, as compared to a reference sequence (e.g., a source nucleic acid). In some embodiments, blocking of 5fC comprises contacting the nucleic acid with sodium borohydride (NaBH4). In some embodiments, blocking of 5fC comprises converting 5fC to a 5fC variant, including one or more of an alcohol, imine, oxime, and/or hydrazone, among other things. In some embodiments, blocking of 5fC may be reversible.

### Example 10: Exemplary Detection of 5fC (not according to the claimed invention)

In some embodiments, the disclosure provides a method for identifying 5fC in a nucleic acid sample comprising steps of:
a. providing a source nucleic acid;
b. blocking a 5caC in the source nucleic acid, e.g. by adding a blocking group;
c. converting 5fC to DHU to provide a processed nucleic acid;
d. optionally amplifying the copy number of the processed nucleic acid;
e. detecting the sequence of the processed nucleic acid;
wherein a C to T transition in the sequence of the processed nucleic acid compared to the source nucleic acid provides the location of 5fC in the source nucleic acid.

In some embodiments, a method for identifying aSfC in the target nucleic acid provides a quantitative measure for frequency of 5fC modifications at each location in a source nucleic acid. In some embodiments, percentages of T at each transition location provide a quantitative level of 5fC at each location in a source nucleic acid.

In some embodiments, adding a blocking group to 5caC in a nucleic acid can be accomplished by (i) contacting the nucleic acid sample with a coupling agent, for example a carboxylic acid derivatization reagent like carbodiimide derivatives such as 1-ethyl-3-(3-dimethylaminopropyl)carbodiimide (EDC) or N,N'-dicyclohexylcarbodiimide (DCC) and (ii) contacting the nucleic acid sample with an amine, hydrazine or hydroxylamine compound. For example, in some embodiments 5caC can be blocked by treating a nucleic acid with EDC and then benzylamine, ethylamine or other amine to form an amide that blocks 5caC from conversion to DHU by, e.g., pic-BH3. Methods for EDC-catalyzed 5caC coupling are described in WO2014165770. In some embodiments, blocking of 5caC can comprise protection or derivatization through a chemical reaction. In some embodiments, blocking of 5caC in a nucleic acid comprises contacting the nucleic acid with a reducing agent and converting 5caC to 5hmC. In some embodiments, subsequent borane reduction, amplification, and/or sequencing of a nucleic acid would produce a C to T transition only for SfC, as compared to a reference sequence. In some embodiments, blocking of 5caC comprises converting 5caC to a 5caC variant, including one or more of an acyl halide, acid anhydride, ester, and/or amide, among other things. In some embodiments, blocking of 5caC may be reversible.

## Claims

1. A method comprising steps of:
a. contacting a source nucleic acid with a metal (VI) oxo complex to produce an oxidized nucleic acid; and
b. contacting the oxidized nucleic acid with a reducing agent to produce a processed nucleic acid;
wherein 5-hydroxymethylcytosine (5hmC) bases in the source nucleic acid are converted to dihydrouracil (DHU) in the processed nucleic acid; and
further comprising one or more additional steps of contacting the oxidized nucleic acid with a metal (VI) oxo complex.

2. The method of claim 1, wherein the source nucleic acid is a mammalian nucleic acid; and/or,
wherein the method does not comprise treatment with bisulfite.

3. The method of any one of the above claims, wherein the 5hmC bases are detected through sequencing; preferably,
wherein the false positive rate for detection of 5hmC is below 1%.

4. The method of any one of the above claims, wherein one or more steps are conducted at a temperature above 4°C; and/or,
wherein the efficiency of conversion of 5hmC to DHU is greater than 80%; and/or,
wherein no affinity enrichment is performed after contacting the source nucleic acid with the metal (VI) oxo complex and before contacting the oxidized nucleic acid with the reducing agent; and/or,
wherein no affinity enrichment is performed.

5. A method comprising steps of:
a. providing a nucleic acid sample comprising a source nucleic acid;
b. modifying the source nucleic acid, comprising steps of:
i. converting the 5hmC in the source nucleic acid to 5-formylcytosine (5fC) and/or 5-carboxylcytosine (5caC) by contacting the source nucleic acid with a metal (VI) oxo complex to produce a processed nucleic acid; and
ii. converting the 5fC and/or 5caC in the processed nucleic acid to DHU;
and further comprising one or more additional steps of contacting the oxidized nucleic acid with a metal (VI) oxo complex;
and
c. detecting the sequence of the processed nucleic acid, comprising one or more of:
i. detecting the presence of DHU in the sequence; and
ii. converting the DHU to thymine (T) and detecting the presence of the thymine (T) in the sequence;
wherein a cytosine (C) to DHU transition or a cytosine (C) to thymine (T) transition in the sequence of the processed nucleic acid compared to the source nucleic acid provides the location of 5hmC in the source nucleic acid.

6. The method of claim 5, wherein the percentages of a DHU or T at each transition location provide a quantitative level of 5hmC at each location in the source nucleic acid.

7. The method according to any one of claims 1-6, wherein the method further comprises the step of amplifying the copy number of the processed nucleic acid; preferably,
wherein the step of amplifying the copy number of the processed nucleic acid comprises performing a polymerase chain reaction (PCR) or primer extension.

8. The method according to any one of claims 5-7, wherein the step of converting the 5caC and/or 5fC to DHU comprises contacting the source nucleic acid with a reducing agent.

9. The method of any one of claims 1-4 and 7-8, wherein the reducing agent is a borane reducing agent; preferably,
wherein the reducing agent is selected from the group consisting of pyridine borane, 2-picoline borane (pic-BH₃), borane, sodium borohydride, sodium cyanoborohydride, and sodium triacetoxyborohydride; preferably,
wherein the reducing agent is pyridine borane or 2-picoline borane.

10. The method according to any one of claims 5-9, wherein the step of detecting the sequence of the processed nucleic acid comprises one or more of chain termination sequencing, microarray, high-throughput sequencing, and restriction enzyme analysis.

11. The method of any one of claims 1-10, wherein the source nucleic acid is provided as part of a sample; preferably,
wherein the sample is a derived from a human.

12. The method of any one of claims 1-11, wherein the source nucleic acid is DNA; preferably,
wherein the source nucleic acid is genomic DNA; and/or,
wherein the source nucleic acid is RNA.

## Patentansprüche

1. Verfahren, umfassend die folgenden Schritte:
a. Inkontaktbringen einer Quellnukleinsäure mit einem Metall(VI)-Oxokomplex, um eine oxidierte Nukleinsäure zu erstellen; und
b. Inkontaktbringen der oxidierten Nukleinsäure mit einem Reduktionsmittel, um eine prozessierte Nukleinsäure zu erstellen;
wobei die Basen von 5-Hydroxymethylcytosin (5hmC) in der Quellnukleinsäure zu Dihydrouracil (DHU) in der verarbeiteten Nukleinsäure umgewandelt werden; und
ferner umfassend einen oder mehrere zusätzliche Schritte des Inkontaktbringens der oxidierten Nukleinsäure mit einem Metall(VI)-Oxokomplex.

2. Verfahren nach Anspruch 1, wobei die Quellnukleinsäure eine Säugetiernukleinsäure ist; und/oder
wobei das Verfahren nicht die Behandlung mit Bisulfit umfasst.

3. Verfahren nach einem der vorstehenden Ansprüche, wobei die 5 hmC Basen durch Sequenzierung nachgewiesen werden; vorzugsweise
wobei die Falsch-Positiv-Rate für den Nachweis von 5 hmC unter 1 % liegt.

4. Verfahren nach einem der vorstehenden Ansprüche, wobei ein oder mehrere Schritte bei einer Temperatur von über 4 °C durchgeführt werden; und/oder
wobei die Effizienz der Umwandlung von 5 hmC in DHU größer als 80 % ist; und/oder
wobei nach dem Inkontaktbringen der Quellnukleinsäure mit dem Metall(VI)-Oxokomplex und vor dem Inkontaktbringen der oxidierten Nukleinsäure mit dem Reduktionsmittel keine Affinitätsanreicherung durchgeführt wird; und/oder
wobei keine Affinitätsanreicherung durchgeführt wird.

5. Verfahren, umfassend die folgenden Schritte:
a. Bereitstellen einer Nukleinsäureprobe, umfassend eine Quellnukleinsäure;
b. Modifizieren der Quellnukleinsäure, umfassend die folgenden Schritte:
i. Umwandeln des 5hmC in der Quellnukleinsäure in 5-Formylcytosin (5fC) und/oder 5-Carboxylcytosin (5caC) durch Inkontaktbringen der Quellnukleinsäure mit einem Metall(VI)-Oxokomplex, um eine prozessierte Nukleinsäure zu erstellen; und
ii. Umwandeln der 5fC und/oder 5caC in der verarbeiteten Nukleinsäure in DHU;
und ferner umfassend einen oder mehrere zusätzliche Schritte des Inkontaktbringens der oxidierten Nukleinsäure mit einem Metall(VI)-Oxokomplex;
und
c. Nachweisen der Sequenz der verarbeiteten Nukleinsäure, umfassend eines oder mehrere von Folgendem:
i. Nachweisen des Vorhandenseins von DHU in der Sequenz; und
ii. Umwandeln des DHU in Thymin (T) und Nachweisen des Vorhandenseins von Thymin (T) in der Sequenz;
wobei ein Cytosin (C)-zu-DHU-Übergang oder ein Cytosin (C)-zu-Thymin (T)-Übergang in der Sequenz der verarbeiteten Nukleinsäure im Vergleich zur Quellnukleinsäure den Ort von 5hmC in der Quellnukleinsäure bereitstellt.

6. Verfahren nach Anspruch 5, wobei die Prozentsätze von DHU oder T an jeder Übergangsstelle eine quantitative Stufe von 5hmC an jeder Stelle in der Quellnukleinsäure bereitstellen.

7. Verfahren nach einem der Ansprüche 1 bis 6, wobei das Verfahren ferner den Schritt der Amplifikation der Kopienzahl der verarbeiteten Nukleinsäure umfasst; vorzugsweise
wobei der Schritt der Amplifikation der Kopienzahl der verarbeiteten Nukleinsäure die Durchführung einer Polymerasekettenreaktion (PCR) oder einer Primerverlängerung umfasst.

8. Verfahren nach einem der Ansprüche 5 bis 7, wobei der Schritt der Umwandlung von 5caC und/oder 5fC in DHU das Inkontaktbringen der Quellnukleinsäure mit einem Reduktionsmittel umfasst.

9. Verfahren nach einem der Ansprüche 1 bis 4 und 7 bis 8, wobei das Reduktionsmittel ein Boran-Reduktionsmittel ist; vorzugsweise
wobei das Reduktionsmittel ausgewählt ist aus der Gruppe, bestehend aus Pyridinboran, 2-Picolinboran (pic-BH₃, Boran, Natriumborhydrid, Natriumcyanoborhydrid und Natriumtriacetoxyborhydrid; vorzugsweise
wobei das Reduktionsmittel Pyridinboran oder 2-Picolinboran ist.

10. Verfahren nach einem der Ansprüche 5 bis 9, wobei der Schritt des Nachweises der Sequenz der verarbeiteten Nukleinsäure eines oder mehrere der Verfahren Kettenterminationssequenzierung, Microarray, Hochdurchsatzsequenzierung und Restriktionsenzymanalyse umfasst.

11. Verfahren nach einem der Ansprüche 1 bis 10, wobei die Quellnukleinsäure als Teil einer Probe bereitgestellt wird; vorzugsweise
wobei die Probe von einem Menschen stammt.

12. Verfahren nach einem der Ansprüche 1 bis 11, wobei die Quellnukleinsäure DNA ist; vorzugsweise
wobei die Quellnukleinsäure genomische DNA ist; und/oder
wobei die Quellnukleinsäure RNA ist.

## Revendications

1. Procédé comprenant les étapes consistant à :
a. mettre en contact un acide nucléique source avec un complexe oxo de métal (VI) pour produire un acide nucléique oxydé ; et
b. mettre en contact l'acide nucléique oxydé avec un agent réducteur pour produire un acide nucléique traité ;
dans lequel les bases 5-hydroxyméthylcytosine (5hmC) de l'acide nucléique source sont converties en dihydro-uracile (DHU) dans l'acide nucléique traité ; et
comprenant en outre une ou plusieurs étapes supplémentaires consistant à mettre en contact l'acide nucléique oxydé avec un complexe oxo de métal (VI).

2. Procédé selon la revendication 1, dans lequel l'acide nucléique source est un acide nucléique de mammifère ; et/ou,
dans lequel le procédé ne comprend pas de traitement au bisulfite.

3. Procédé selon l'une quelconque des revendications précédentes, dans lequel les bases 5hmC sont détectées par séquençage ; de préférence,
dans lequel le taux de faux positifs pour la détection de 5hmC est inférieur à 1 %.

4. Procédé selon l'une quelconque des revendications précédentes, dans lequel une ou plusieurs étapes sont conduites à une température supérieure à 4 °C ; et/ou,
dans lequel l'efficacité de conversion de 5hmC en DHU est supérieure à 80 % ; et/ou,
dans lequel aucun enrichissement par affinité n'est effectué après la mise en contact de l'acide nucléique source avec le complexe oxo de métal (VI) et avant la mise en contact de l'acide nucléique oxydé avec l'agent réducteur ; et/ou,
dans lequel aucun enrichissement par affinité n'est effectué.

5. Procédé comprenant les étapes consistant à :
a. fournir un échantillon d'acide nucléique comprenant un acide nucléique source ;
b. modifier l'acide nucléique source, comprenant les étapes consistant à :
i. convertir la 5hmC de l'acide nucléique source en 5-formylcytosine (5fC) et/ou en 5-carboxylcytosine (5caC) en mettant en contact l'acide nucléique source avec un complexe oxo de métal (VI) afin de produire un acide nucléique traité ; et
ii. convertir la 5fC et/ou la 5caC de l'acide nucléique traité en DHU ;
et comprenant en outre une ou plusieurs étapes supplémentaires consistant à mettre en contact l'acide nucléique oxydé avec un complexe oxo de métal (VI) ;
et
c. détecter la séquence de l'acide nucléique traité, comprenant une ou plusieurs parmi :
i. la détection de la présence de DHU dans la séquence ; et
ii. la conversion de la DHU en thymine (T) et la détection de la présence de la thymine (T) dans la séquence ;
dans lequel une transition de cytosine (C) vers DHU ou une transition de cytosine (C) vers thymine (T) dans la séquence de l'acide nucléique traité par rapport à l'acide nucléique source fournit l'emplacement de 5hmC dans l'acide nucléique source.

6. Procédé selon la revendication 5, dans lequel les pourcentages d'une DHU ou d'une T à chaque emplacement de transition fournissent un niveau quantitatif de 5hmC à chaque emplacement dans l'acide nucléique source.

7. Procédé selon l'une quelconque des revendications 1 à 6, dans lequel le procédé comprend en outre l'étape consistant à amplifier le nombre de copies de l'acide nucléique traité ; de préférence,
dans lequel l'étape consistant à amplifier le nombre de copies de l'acide nucléique traité comprend la réalisation d'une réaction en chaîne par polymérase (PCR) ou d'une extension d'amorce.

8. Procédé selon l'une quelconque des revendications 5 à 7, dans lequel l'étape consistant à convertir la 5caC et/ou 5fC en DHU comprend la mise en contact de l'acide nucléique source avec un agent réducteur.

9. Procédé selon l'une quelconque des revendications 1 à 4 et 7 à 8, dans lequel l'agent réducteur est un agent réducteur au borane ; de préférence,
dans lequel l'agent réducteur est choisi dans le groupe constitué de pyridine borane, 2-picoline borane (pic-BH₃), borane, borohydrure de sodium, cyanoborohydrure de sodium, et triacétoxyborohydrure de sodium ; de préférence,
dans lequel l'agent réducteur est le pyridine borane ou le 2-picoline borane.

10. Procédé selon l'une quelconque des revendications 5 à 9, dans lequel l'étape consistant à détecter la séquence de l'acide nucléique traité comprend un ou plusieurs parmi un séquençage par terminaison de chaîne, un microréseau, un séquençage à haut débit, et une analyse des enzymes de restriction.

11. Procédé selon l'une quelconque des revendications 1 à 10, dans lequel l'acide nucléique source est fourni en tant que partie d'un échantillon ; de préférence,
dans lequel l'échantillon est issu d'un être humain.

12. Procédé selon l'une quelconque des revendications 1 à 11, dans lequel l'acide nucléique source est de l'ADN ; de préférence,
dans lequel l'acide nucléique source est de l'ADN génomique ; et/ou,
dans lequel l'acide nucléique source est de l'ARN.
